(19) Europäisches Patentamt
European Patent Office
Office européen des brevets

(11) Numéro de publication: **0 220 118 B1**

(12) **FASCICULE DE BREVET EUROPEEN**

(45) Date de publication de fascicule du brevet: **02.01.92**

(51) Int. Cl.[5]: **C07C 65/36**, C07C 65/17, C07C 69/76, C07C 233/64, C07C 33/34, C07C 47/445

(21) Numéro de dépôt: **86402257.9**

(22) Date de dépôt: **10.10.86**

(54) **Dérivés bicycliques naphtaléniques, leur procédé de préparation et leur utilisation en médecine humaine et vétérinaire et en cosmétique.**

(30) Priorité: **11.10.85 FR 8515106**
**09.07.86 FR 8610020**

(43) Date de publication de la demande:
**29.04.87 Bulletin 87/18**

(45) Mention de la délivrance du brevet:
**02.01.92 Bulletin 92/01**

(84) Etats contractants désignés:
**AT BE CH DE ES FR GB GR IT LI NL SE**

(56) Documents cités:
**FR-A- 2 555 571**
**FR-A- 2 573 755**
**US-A- 1 917 285**
**US-A- 4 454 341**

(73) Titulaire: **CENTRE INTERNATIONAL DE RECHERCHES DERMATOLOGIOUES GALDERMA - CIRD GALDERMA**
**Sophia Antipolis**
**F-06560 Valbonne(FR)**

(72) Inventeur: **Maignan, Jean**
**8, rue Halevy**
**F-93290 Tremblay les Gonesse(FR)**
Inventeur: **Lang, Gérard**
**44, Avenue Lacour**
**F-95210 Saint Gratien(FR)**
Inventeur: **Malle, Gérard**
**18, Grande rue**
**F-77580 Villiers sur Morin(FR)**
Inventeur: **Restle, Serge**
**140, rue Anatole France**
**F-93600 Aulnay-Sous-Bois(FR)**
Inventeur: **Shroot, Braham Villa 35**
**Hameaux de Val Bosquet Chemin de Val Bosquet**
**F-06600 Antibes(FR)**

(74) Mandataire: **Nony, Michel**
**Cabinet NONY & CIE, 29, rue Cambacérès**
**F-75008 Paris(FR)**

EP 0 220 118 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet de nouveaux dérivés bicycliques naphtaléniques, leur procédé de préparation et leur utilisation en médecine humaine et vétérinaire et en cosmétique.

Les composés selon l'invention présentent une activité dans le traitement topique et systémique des affections dermatologiques liées à un désordre de la kératinisation (différenciation-prolifération) et d'affections dermatologiques (ou autres) à composante inflammatoire et/ou immunoallergique et dans le traitement des maladies de dégénérescence du tissu conjonctif ainsi qu'une activité anti-tumorale.

En outre, ces composés peuvent être utilisés dans le traitement de l'atopie qu'elle soit cutanée ou respiratoire et du psoriasis rhumatoïde.

Ces composés possèdent également une bonne activité sur les germes impliqués dans l'acné.

Enfin, ils trouvent une application dans le domaine ophtalmologique notamment dans le traitement des cornéopathies.

L'état de la technique est représenté essentiellement par le brevet américain n° 4.454.341 ainsi que par les demandes de brevet français FR 2.555.571 et FR 2.573.755.

Les dérivés bicycliques naphtaléniques selon l'invention peuvent être représentés par la formule générale suivante :

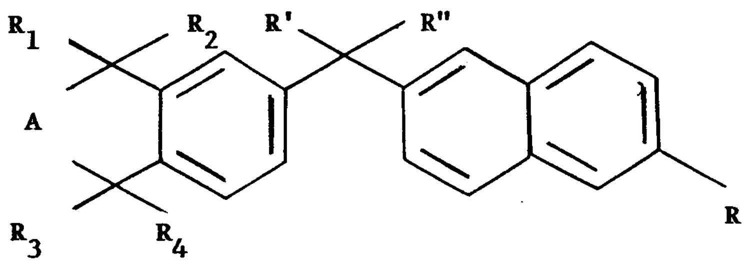

(I)

dans laquelle :

A représente un radical méthylène ou diméthylène, substitués ou non par un radical alkyle inférieur,

$R_1$, $R_2$, $R_3$ et $R_4$, identiques ou différents, représentent un atome d'hydrogène ou un radical alkyle inférieur ayant de 1 à 6 atomes de carbone,

ou $R_1$ et $R_3$, pris ensemble, forment un pont méthylène ou diméthylène, quand A représente un radical diméthylène,

R' représente un atome d'hydrogène, un radical OH, un radical alkoxy ayant de 1 à 4 atomes de carbone, un radical acyloxy ayant de 1 à 4 atomes de carbone ou un radical amino,

R'' représente un atome d'hydrogène ou un radical alkoxy ayant de 1 à 4 atomes de carbone,

ou R' et R'', pris ensemble, forment un radical oxo (=O), méthano ($=CH_2$) ou hydroxyimino (=N-OH),

R représente le radical $-CH_2OH$ ou le radical $-COR_5$,

$R_5$ représentant un atome d'hydrogène, le radical $OR_6$ ou le radical

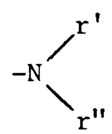

$R_6$ représentant un atome d'hydrogène, un radical alkyle ayant de 1 à 20 atomes de carbone, monohydroxyalkyle, polyhydroxyalkyle, aryle ou aralkyle éventuellement substitué(s) où un reste d'un sucre ou encore le radical

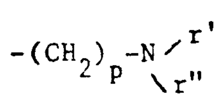

p étant 1, 2 ou 3 et r' et r'', identiques ou différents, représentant un atome d'hydrogène, un radical alkyle inférieur, un radical monohydroxyalkyle éventuellement interrompu par un hétéroatome, un radical polyhydroxyalkyle, un radical aryle ou benzyle éventuellement substitué(s), un reste d'aminoacide, d'aminoester ou de sucre aminé ou pris ensemble forment, avec l'atome d'azote auquel ils sont rattachés, un hétérocycle substitué ou non,

et les sels desdits composés de formule (I) ainsi que leurs isomères optiques.

Par radical alkyle inférieur on doit entendre un radical ayant de 1 à 6 atomes de carbone.

Parmi les radicaux alkyles inférieurs et ceux ayant jusqu'à 20 atomes de carbone on peut citer les radicaux méthyle, éthyle, propyle, isopropyle, butyle, tertiobutyle, éthyl-2 hexyle, octyle, isooctyle, dodécyle, hexadécyle et octadécyle.

Par radical monohydroxyalkyle on doit entendre un radical ayant 2 à 6 atomes de carbone notamment un radical hydroxy-2 éthyle, hydroxy-2 propyle ou hydroxy-2' éthoxy-2 éthyle.

Par radical polyhydroxyalkyle, on doit entendre un radical contenant de 3 à 6 atomes de carbone et de 2 à 5 groupes hydroxyles tels que les radicaux dihydroxy-2,3 propyle, dihydroxy-1,3 propyl-2 ou le reste du pentaérythritol.

Comme radical alkoxy ayant de 1 à 4 atomes de carbone on peut citer le radical méthoxy, isopropoxy, butoxy ou tert-butoxy.

Par radical aryle, on doit entendre un radical phényle éventuellement substitué par au moins un atome d'halogène, -OH, $-NO_2$ , un radical alkyle inférieur, un radical trifluorométhyle ou une fonction acide carboxylique.

Comme radical aralkyle préféré on peut citer le radical benzyle ainsi que le radical phénéthyle.

Par reste d'un sucre, on doit entendre un reste dérivant par exemple du glucose, du mannose, de l'érythrose ou du galactose.

Parmi les restes de sucres aminés on peut citer ceux dérivant de glucosamine, de galactosamine, de mannosamine ou de méglumine.

Lorsque les radicaux r' et r'' pris ensemble forment, avec l'atome d'azote auquel il sont rattachés, un hétérocycle, celui-ci est de préférence un radical pipéridino, pipérazino, morpholino, pyrrolidino ou (hydroxy-2' éthyl)-4 pipérazino.

Quand les composés selon l'invention se présentent sous forme de sels, il peut s'agir soit de sels d'un métal alcalin ou alcalino-terreux ou encore de zinc, ou d'une amine organique lorsqu'ils comportent au moins une fonction acide libre, soit de sels d'un acide minéral ou organique notamment de chlorhydrate, de bromhydrate ou de citrate lorsqu'ils comportent au moins une fonction amine.

Parmi les composés de formule (I) particulièrement préférés selon l'invention, on peut mentionner ceux correspondant aux formules II, III et IV suivantes:

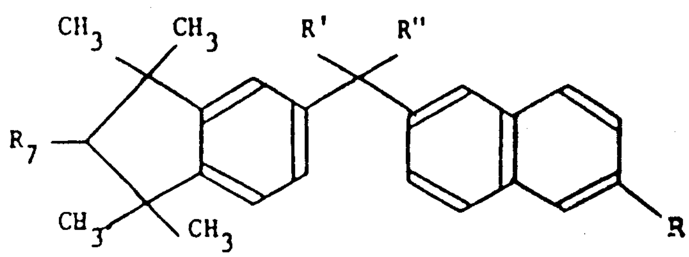

(II)

dans laquelle:

R' représente un atome d'hydrogène ou un radical OH,

R'' représente un atome d'hydrogène,

ou R' et R'' pris ensemble forment un radical oxo (=O),

R représente le radical $CH_2OH$ ou le radical $-COOR_6$,

$R_6$ représentant un atome d'hydrogène ou un radical alkyle ayant de 1 à 6 atomes de carbone,

et $R_7$ représente un atome d'hydrogène ou un radical méthyle.

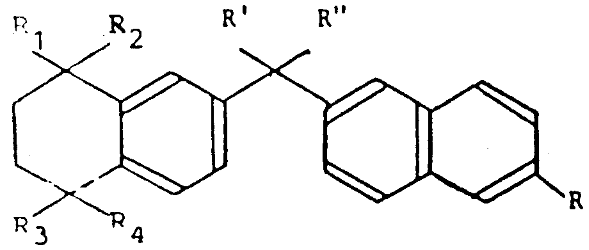

(III)

dans laquelle:

$R_1$, $R_2$, $R_3$ et $R_4$ représentent le radical $-CH_3$,

R' représente un atome d'hydrogène, un radical OH, un radical alkoxy ayant de 1 à 4 atomes de carbone, un radical acyloxy ayant de 1 à 4 atomes de carbone,

R" représente un atome d'hydrogène,

ou R' et R" pris ensemble forment un radical oxo (=O) ou un radical méthano ($=CH_2$),

et R représente le radical $-CH_2OH$ ou le radical $-COR_5$,

$R_5$ représentant un atome d'hydrogène, le radical-$OR_6$ ou le radical

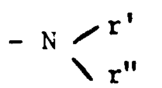 ,

$R_6$ représentant un atome d'hydrogène ou un radical alkyle ayant de 1 à 6 atomes de carbone,

et r' ou r" représentant un atome d'hydrogène, un radical alkyle ayant de 1 à 6 atomes de carbone, hydroxy-4 phényle, hydroxy-2'éthoxy éthyle ou carboxy-1 méthylthio-3 propyle.

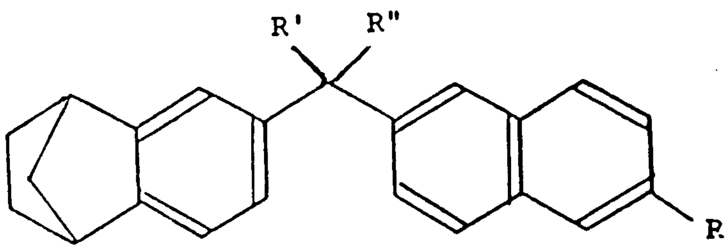

(IV)

dans laquelle:

R' et R" pris ensemble forment un radical oxo (=O),

R représente $-COR_5$ ,

$R_5$ représentant le radical $-OR_6$ ou le radical

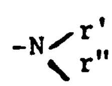

$R_6$ représentant un atome d'hydrogène ou un radical alkyle ayant de 1 à 6 atomes de carbone,

et r' et r" représentant un atome d'hydrogène ou un radical alkyle ayant de 1 à 6 atomes de carbone.

Parmi les composés de formule (I) selon l'invention on peut notamment citer:

1) [(tétraméthyl-1,1,3,3 indanyl-5) carbonyl]-6 naphtalène carboxylate de méthyle-2,
2) Acide [(tétraméthyl-1,1,3,3 indanyl-5) carbonyl]-6 naphtalène carboxylique-2,
3) [(pentaméthyl-1,1,2,3,3 indanyl-5) carbonyl]-6 naphtalène carboxylate de méthyle-2,
4) Acide [(pentaméthyl-1,1,2,3,3 indanyl-5) carbonyl]-6 naphtalène carboxylique-2,
5) Acide [(pentaméthyl-1,1,2,3,3 indanyl-5) hydroxyméthyl]-6 naphtalène carboxylique-2,
6) Acide [tétraméthyl-5,5,8,8 tétrahydro-5,6,7,8 naphtyl-2) hydroxyméthyl]-6 naphtalène carboxylique-2,
7) N-éthyl [(tétraméthyl-5,5,8,8 tétrahydro-5,6,7,8 naphtyl-2) hydroxyméthyl]-6 naphtalène carboxamide-2,
8) (tétraméthyl-5,5,8,8 tétrahydro-5,6,7,8 naphtyl-2)-1 (carboxy-6 naphtyl -2)-1 méthane,
9) N-éthyl [tétraméthyl-1,1,3,3 indanyl-5) carbonyl]-6 naphtalène carboxamide-2,
10) N-éthyl [(pentaméthyl-1,1,2,3,3 indanyl-5) carbonyl]-6 naphtalène carboxamide-2,

11) N-éthyl [(pentaméthyl-1,1,2,3,3 indanyl-5) hydroxyméthyl]-6 naphtalène carboxamide-2,
12) Acide [(tétraméthyl-5,5,8,8 tétrahydro-5,6,7,8 naphtyl-2) acétoxyméthyl]-6 naphtalène carboxylique-2,
13) [(tétraméthyl-5,5,8,8 tétrahydro-5,6,7,8 naphtyl-2)-2 éthényl-2] -6 naphtalène carboxylate de méthyle-2,
14) Acide [tétraméthyl-5,5,8,8 tétrahydro-5,6,7,8 naphtyl-2)-2 éthényl-2]-6 naphtalène carboxylique-2,
15) [(tétraméthyl-5,5,8,8 tétrahydro-5,6,7,8 naphtyl-2) hydroxyméthyl]-6 naphtalène carbinol-2,
16) [(tétraméthyl-5,5,8,8 tétrahydro-5,6,7,8 naphtyl-2) butoxyméthyl]-6 naphtalène carbinol-2,
17) Acide [(tétraméthyl-5,5,8,8 tétrahydro-5,6,7,8 naphtyl-2) butoxyméthyl]-6 naphtalène carboxylique-2,
18) [(tétraméthyl-5,5,8,8 tétrahydro-5,6,7,8 naphtyl-2) hydroxyméthyl]-6 naphtaldéhyde-2,
19) [(tétraméthyl-5,5,8,8 tétrahydro-5,6,7,8 naphtyl-2) hydroxyiminométhyl]-6 naphtalène carboxylate d'éthyle-2,
20) Acide [(tétraméthyl-5,5,8,8 tétrahydro-5,6,7,8 naphtyl-2) hydroxyiminométhyl]-6 naphtalène carboxylique-2,
21) N-éthyl [(tétraméthyl-5,5,8,8 tétrahydro-5,6,7,8 naphtyl-2) hydroxyiminométhyl]-6 naphtalène carboxamide-2,
22) [(tétraméthyl-5,5,8,8 tétrahydro-5,6,7,8 naphtyl-2) aminométhyl]-6 naphtalène carboxylate d'éthyle-2,
23) Acide [(tétraméthyl-5,5,8,8 tétrahydro-5,6,7,8 naphtyl-2) aminométhyl]-6 naphtalène carboxylique-2,
24) Acide [(tétraméthyl-5,5,8,8 tétrahydro-5,6,7,8 naphtyl-2) carbonyl]-6 naphtalène carboxylique-2 et son ester méthylique
25) N-éthyl [(tétraméthyl-5,5,8,8 tétrahydro-5,6,7,8 naphtyl-2) carbonyl]-6 naphtalène carboxamide-2,
26) [(tétraméthyl-5,5,8,8 tétrahydro-5,6,7,8 naphtyl-2) carbonyl]-6 naphtalène carboxaldéhyde-2,
27) [(tétraméthyl-5,5,8,8 tétrahydro-5,6,7,8 naphtyl-2) carbonyl]-6 naphtalène carbinol-2,
28) N (-hydroxy-2' éthoxy-2 éthyl) [(tétraméthyl-5,5,8,8 tétrahydro-5,6,7,8 naphtyl-2) carbonyl]-6 naphtalène carboxamide-2,
29) N-p-hydroxyphényl [(tétraméthyl-5,5,8,8 tétrahydro-5,6,7,8 naphtyl-2) carbonyl]-6 naphtalène carboxamide-2,
30) N-(éthoxycarbonyl-1 méthylthio-3 propyl) [(tétraméthyl-5,5,8,8 tétrahydro-5,6,7,8 naphtyl-2) carbonyl]-6 naphtalène carboxamide-2,
31) N-(carboxy-1 méthylthio-3 propyl) [(tétraméthyl-5,5,8,8 tétrahydro-5,6,7,8 naphtyl-2) carbonyl]-6 naphtalène carboxamide-2,
32) Acide [(méthano-5,8 tétrahydro-5,6,7,8 naphtyl-2) carbonyl]-6 naphtalène carboxylique-2 et son ester méthylique,
33) Acide [(tétrahydro-5,6,7,8 naphtyl-2) carbonyl]-6 naphtalène carboxylique-2 et son ester méthylique,
34) N-éthyl [(tétrahydro-5,6,7,8 naphtyl-2) carbonyl]-6 naphtalène carboxamide-2.

La présente invention a également pour objet le procédé de préparation des composés de formule (I).

Ces composés peuvent être obtenus selon différentes méthodes en fonction de leur structure, ils sont de préférence obtenus selon le schéma réactionnel suivant:

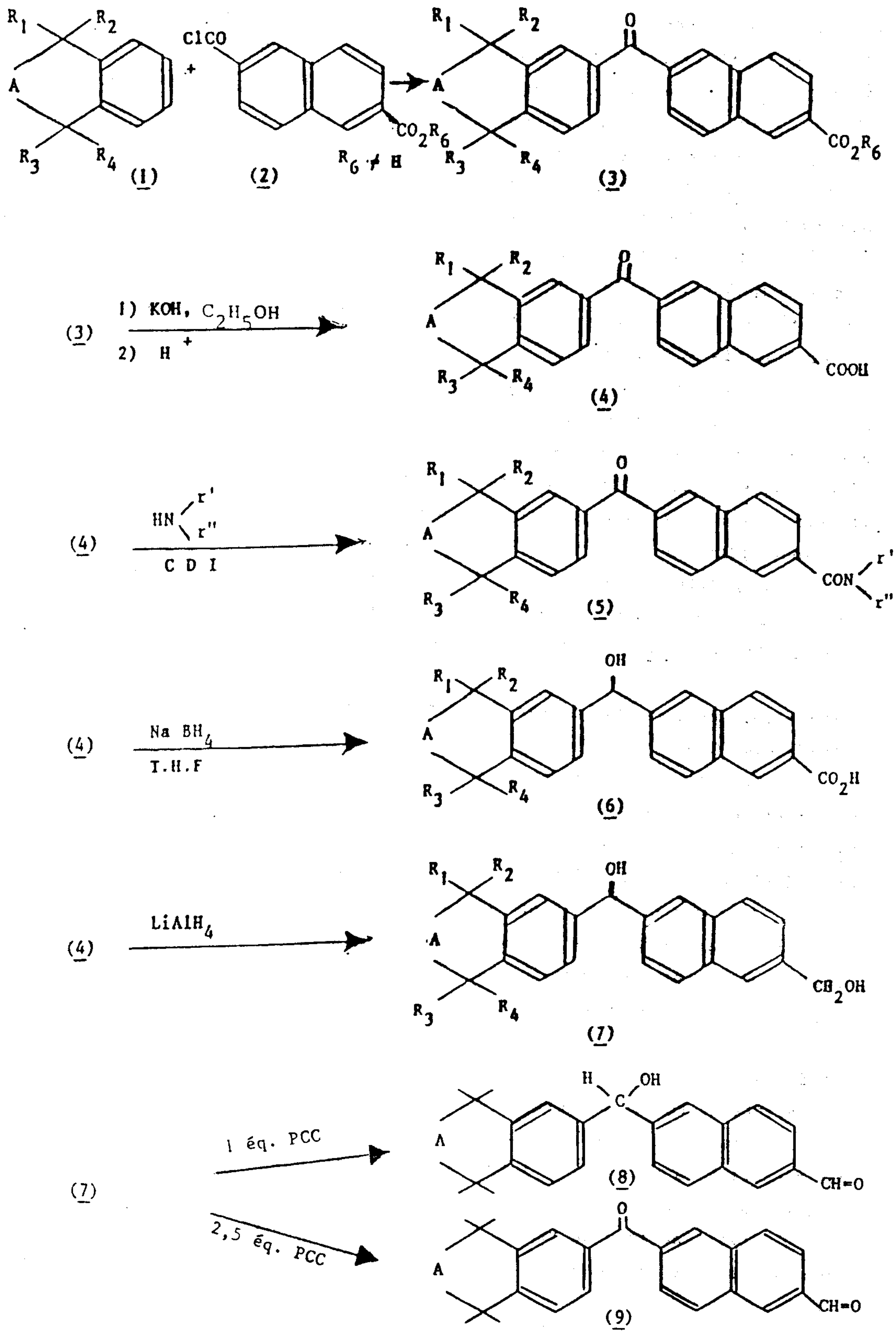

(1) (2) (3)

(3) (4)

(4) (5)

(4) (6)

(4) (7)

(7) (8) (9)

Le chlorure d'acide alkoxycarbonyl-6 naphtalène carboxylique-2 (2) est obtenu par réaction de monosaponification d'un naphtalène dicarboxylate d'alkyl-2,6 et formation du chlorure d'acide par action de chlorure de thionyle selon la méthode classique de préparation des chlorures d'acides.

Parmi les produits de départ de formule (1) la tétraline et l'indane sont des produits commerciaux. Le tétraméthyl-5,5,8,8 tétrahydro-5,6,7,8 naphtalène (ou tétraméthyl-5,5,8,8 tétraline) est préparé suivant la

méthode décrite par H.Q BRUSON ET J.W. KROGER, J. Am. Chem. Soc., 62 36-44 (1940). Le méthano-5,8 tétrahydro-5,6,7,8 naphtalène est obtenu selon la méthode décrite dans J. Org. Chem. 32, 893-901 (1967). Le pentaméthyl-1,1,2,3,3 indane et le tétraméthyl-1,1,3,3 indane sont obtenus selon les méthodes décrites dans le brevet français 1.392.804.

La réaction de condensation du chlorure d'acide alkoxycarbonyl-6 naphtalène carboxylique-2 (2) sur le composé aromatique bicyclique (1) est effectuée dans les conditions habituelles de la réaction de Friedel-Crafts, c'est-à-dire en présence de chlorure d'aluminium ou de chlorure stanneux anhydre dans du dichloro-1,2 éthane à une température comprise entre 0° et 25° C sous agitation.

A partir du céto-ester (3) on accède par saponification au céto-acide correspondant (4) qui peut ensuite être transformé en amide de formule (5) par action d'une amine de formule

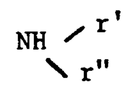

(r' et r" ayant les mêmes significations que données ci-dessus) en présence de N,N'-carbonyl diimidazole (CDI).

Lorsque $R_6$ représente un radical monohydroxy ou polyhydroxyalkyle, il est préférable de préparer le céto-acide (4) à partir de l'ester méthylique (3) ($R_6 = -CH_3$) et ensuite d'estérifier le céto-acide ainsi obtenu en céto-ester de l'alcool mono ou polyhydrique choisi selon les méthodes connues.

A partir du céto-acide (4) la réduction par le borohydrure de sodium dans un solvant organique tel que le T.H.F permet d'obtenir l'alcool secondaire (6) et la réduction par l'hydrure de lithium aluminium du céto-acide (4) permet d'accéder au diol (7).

Par oxydation du diol (7) par un équivalent de chlorochromate de pyridinium (PCC) on accède à l'alcool aldéhyde (8) ; avec au moins 2 équivalents de PCC on obtient le cétoaldéhyde (9).

Les composés selon l'invention dans lesquels R' = R" = H sont obtenus par réduction au zinc des dérivés cétoniques dans l'acide acétique en présence d'acide chlorhydrique.

Les réactions de réduction du carbonyle doivent bien entendu être compatibles avec la nature du groupement R. Il peut être souhaitable d'en assurer la protection éventuelle, toutefois la réduction du carbonyle ne soulève aucune difficulté lorsque $R = CO_2H$.

Les dérivés acyloxy des composés de formule (I) (R' = acyloxy $C_1$-$C_4$ et R" = H) sont obtenus en faisant réagir une forme activée d'acide, tel qu'un anhydride ou un chlorure d'acide sur un composé selon l'invention dans lequel: R' = OH et R" = H.

Les dérivés alkoxy des composés de formule (I) (R' = alkoxy $C_1$-$C_4$ et R" = H) sont de même obtenus à partir des composés de formule (I) (R' = OH et R" = H) selon les méthodes connues.

Pour la préparation des dérivés acyloxy et alkoxy, il est préférable que le radical R soit une fonction ester, acide ou amide.

Les composés de formule (I) dans laquelle R' et R" = méthano ($CH_2 =$) sont obtenus par réaction de Wittig selon le schéma réactionnel suivant:

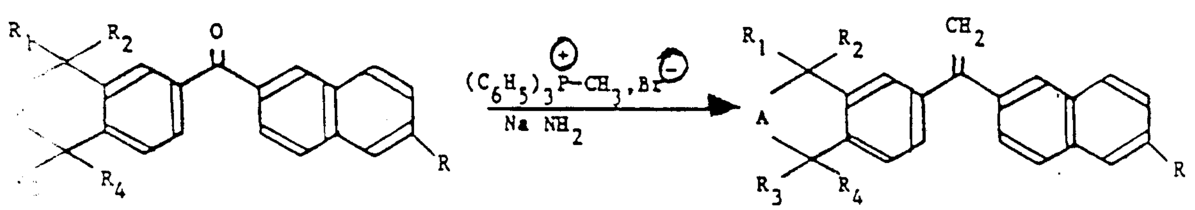


Les composés de formule (I) dans laquelle R' et R" = hydroxyimino (= N-OH) sont obtenus par action du chlorhydrate d'hydroxylamine sur les composés carbonylés correspondants, dans un solvant organique, tel que l'éthanol en présence d'une base minérale telle que le bicarbonate de sodium ou d'une base organique telle que la triéthylamine. Ces dérivés hydroxyimino conduisent par réduction au zinc, en milieu acétique, aux amines de formule (I) dans laquelle R' = $NH_2$ et R" = H.

La présente invention a également pour objet, à titre de médicament, les composés de formule (I) telle que définie ci-dessus.

Ces composés sont actifs dans le test d'inhibition de l'ornithine décarboxylase après induction, par "tape stripping", chez le rat nu M. Bouclier et al, Dermatologica 169 n° 4 (1984). Ce test est admis comme mesure d'une action antiproliférative.

Ces composés conviennent particulièrement bien pour traiter les affections dermatologiques liées à un désordre de la kératinisation (différenciation-prolifération) ainsi que les affections dermatologiques, ou autres, à composante inflammatoire et/ou immunoallergique notamment:

- les acnés vulgaires, comédoniennes ou polymorphes, les acnés séniles, solaires et les acnés médicamenteuses ou professionnelles,
- les formes étendues et/ou sévères de psoriasis, et les autres troubles de la kératinisation, et notamment les ichtyoses et états ichtyosiformes,
- la maladie de Darier,
- les kératodermies palmo-plantaires,
- les leucoplasies et états leucoplasiformes, le lichen plan,
- toutes proliférations dermatologiques bénignes ou malignes, sévères ou étendues.

Ils sont également actifs dans le traitement des tumeurs, du psoriasis rhumatoïde, des atopies cutanées ou respiratoires ainsi que de certains problèmes ophtalmologiques relatifs aux cornéopathies.

Ces composés possèdent également une bonne activité sur les germes impliqués dans l'acné.

La présente invention a donc également pour objet des compositions médicamenteuses contenant au moins un composé de formule (I) tel que défini ci-dessus, ou un de ses sels, ou un de ses isomères optiques.

La présente invention a donc aussi pour objet une nouvelle composition médicamenteuse, destinée notamment au traitement des affections susmentionnées, caractérisée par le fait qu'elle comporte, dans un support pharmaceutique acceptable, au moins un composé de formule (I) et/ou un de ses sels et/ou un de ses isomères optiques.

Les composés selon l'invention sont généralement administrés à une dose journalière d'environ 2µg/kg à 2mg/kg de poids corporel.

Comme support des compositions, on peut utiliser tout support conventionnel, le composé actif se trouvant soit à l'état dissous, soit à l'état dispersé dans le véhicule.

L'administration peut être effectuée par voie entérale, parentérale, topique ou oculaire. Par voie entérale, les médicaments peuvent se présenter sous forme de comprimés, de gélules, de dragées, de sirops, de suspensions, de solutions, de poudres, de granulés, d'émulsions. Par voie parentérale, les compositions peuvent se présenter sous forme de solutions ou suspensions pour perfusion ou pour injection.

Par voie topique, les compositions pharmaceutiques à base des composés selon l'invention se présentent sous forme d'onguents, de teintures, de crèmes, de pommades, de poudres, de timbres, de tampons imbibés, de solutions, de lotions, de gels, de sprays ou encore de suspensions.

Ces compositions par voie topique peuvent se présenter soit sous forme anhydre, soit sous forme aqueuse selon l'indication clinique.

Par voie oculaire, ce sont particulièrement des collyres.

Les compositions par voie topique ou oculaire contiennent de préférence de 0,0005 à environ 5% en poids d'au moins un composé de formule (I) telle que définie ci-dessus, par rapport au poids total de la composition.

Les composés de formule (I) selon l'invention, trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la prévention ou le traitement des effets néfastes du soleil ou dans le traitement des peaux physiologiquement sèches.

La présente invention vise donc également une composition cosmétique contenant, dans un support cosmétiquement acceptable, au moins un composé de formule (I) ou un de ses sels et/ou un de ses isomères, cette composition se présentant notamment sous forme de lotion, gel, crème, savon ou shampooing.

La concentration en composé de formule (I), dans les compositions cosmétiques, est comprise entre 0,0005 et 2% en poids et de préférence entre 0,01 et 1% en poids.

Les compositions médicamenteuses et cosmétiques selon l'invention peuvent contenir des additifs inertes ou mêmes pharmacodynamiquement ou cosmétiquement actifs et notamment des agents hydratants, comme la thiamorpholinone et ses dérivés ou l'urée; des agents antiséborrhéiques ou antiacnéiques, tels que la S-carboxyméthylcystéine, la S-benzylcystéamine, leurs sels et leurs dérivés, la tioxolone ou le peroxyde de benzoyle; des antibiotiques comme l'érythromycine et ses esters, la néomycine, les tétracyclines et les polyméthylène-4,5 isothiazolones-3; des agents favorisant la repousse des cheveux, comme le "Minoxidil" (diamino-2,4 pipéridino-6 pyrimidine oxyde-3) et ses dérivés, le Diazoxide (chloro-7 méthyl-3 benzothiadiazine-1,2,4 dioxyde-1,1) et le Phénytoïn (diphényl-5,5 imidazolidine dione-2,4); des agents anti-inflammatoires stéroïdiens et non stéroïdiens; des caroténoïdes et, notamment le $\beta$-carotène; des agents anti-psoriasiques tels que l'anthraline et ses dérivés et les acides eicosatétraynoïque-5,8,11,14 et triynoïque-5,8,11 ainsi que leurs esters et amides.

Les compositions selon l'invention peuvent également contenir des agents d'amélioration de la saveur, des agents conservateurs, des agents stabilisants, des agents régulateurs d'humidité, des agents régulateurs de pH, des agents modificateurs de pression osmotique, des agents émulsionnants, des filtres UV-A et UV-B, des anti-oxydants tels que de l'$\alpha$-tocophérol, le butylhydroxyanisole ou le butylhydroxytoluène.

On va maintenant donner, à titre d'illustration et sans aucun caractère limitatif, plusieurs exemples de préparation des composés actifs de formule (I) selon l'invention ainsi que des exemples de compositions les contenant.

## EXEMPLE I

Préparation du [(tétraméthyl-1,1,3,3 indanyl-5) carbonyl]-6 naphtalène carboxylate de méthyle-2.

Composé de formule II dans laquelle: R' et R'' = oxo, $R_7$ = H et R = -$CO_2CH_3$.

A une suspension de 2,26g (13 mmoles) de tétraméthyl-1,1,3,3 indane et de 3,23g (13 mmoles) de chlorure d'acide méthoxycarbonyl-6 naphtalène carboxylique-2 dans 80cm$^3$ de dichloro-1,2 éthane anhydre, on ajoute par portions 3,33g (25 mmoles) de chlorure d'aluminium anhydre. Le mélange est agité pendant 5h à température ambiante puis versé dans 100cm$^3$ d'eau glacée. La phase organique est décantée et la phase aqueuse extraite deux fois à l'aide de 100cm$^3$ de dichloroéthane. Les phases de dichloroéthane sont rassemblées, lavées au bicarbonate de sodium, séchées sur sulfate de sodium puis concentrées sous pression réduite. Le solide obtenu est repris par 100cm$^3$ de méthanol, essoré puis recristallisé dans 250cm$^3$ de méthanol. Après filtration et séchage sous vide on obtient 3,4g de paillettes jaune pâle de [(tétraméthyl-1,1,3,3 indanyl-5) carbonyl]-6 naphtalène carboxylate de méthyle-2 de point de fusion: 135 °C.

Le spectre $^1$H R.M.N 60 MHz est conforme à la structure attendue.

| Analyse élémentaire: $C_{26}H_{26}O_3$ | | | | | | |
|---|---|---|---|---|---|---|
| Calculé :<br>Trouvé: | C: | 80,80<br>80,75 | H: | 6,78<br>6,82 | O: | 12,42<br>12,69 |

## EXEMPLE II

Préparation de l'acide [(tétraméthyl-1,1,3,3 indanyl-5) carbonyl]-6 naphtalène carboxylique-2.

Composé de formule II dans laquelle: R' et R'' = oxo, $R_7$ = H et R = -$CO_2H$.

Une suspension de 1,4g (3,6 mmoles) de [(tétraméthyl-1,1,3,3 indanyl-5) carbonyl]-6 naphtalène carboxylate de méthyle-2, obtenu à l'exemple I, est agitée 2h30 dans un mélange de 25cm$^3$ d'alcool et 25cm$^3$ de potasse aqueuse 6N chauffé au reflux. Après addition de 80cm$^3$ d'eau, l'alcool est éliminé par évaporation sous vide. La phase aqueuse obtenue est diluée à 250cm$^3$, refroidie entre 0 et +5 °C puis acidifiée par 20cm$^3$ d'acide chlorhydrique 12N. Le précipité obtenu est essoré, lavé à l'eau et séché sur potasse à 80-100 °C. Après recristallisation dans le méthanol avec traitement au charbon animal, on obtient 1,06g de cristaux blancs d'acide [(tétraméthyl-1,1,3,3 indanyl-5) carbonyl]-6 naphtalène carboxylique-2 de point de fusion: 231-2 °C.

Le spectre $^1$H R.M.N 80 MHz est conforme à la structure attendue.

| Analyse élémentaire: $C_{25}H_{24}O_3$ | | | | | | |
|---|---|---|---|---|---|---|
| Calculé:<br>Trouvé: | C: | 80,62<br>80,71 | H: | 6,50<br>6,44 | O: | 12,89<br>12,84 |

## EXEMPLE III

Préparation du [(pentaméthyl-1,1,2,3,3 indanyl-5) carbonyl]-6 naphtalène carboxylate de méthyle-2.

Composé de formule (II) dans laquelle: R' et R'' = oxo, $R_7$ = $-CH_3$ et R = $-CO_2CH_3$.

A une suspension de 2,47g (13 mmoles) de pentaméthyl-1,1,2,3,3 indane et de 3,23g (13 mmoles) de chlorure d'acide méthoxycarbonyl-6 naphtalène carboxylique-2 dans 80cm$^3$ de dichloro-1,2 éthane anhydre, on ajoute, par portions en 1h, 3,33g (25 mmoles) de chlorure d'aluminium anhydre. Le mélange est agité pendant 5h à température ambiante puis versé dans 100cm$^3$ d'eau glacée. La phase organique est décantée et la phase aqueuse extraite deux fois par 100cm$^3$ de dichloroéthane. Les phases de dichloroéthane sont rassemblées, lavées au bicarbonate de sodium, séchées sur sulfate de sodium puis concentrées sous pression réduite. Le solide obtenu est repris par 60cm$^3$ de méthanol, essoré, séché puis purifié par chromatographie sur gel de silice 60 dans un mélange hexane/toluène/éther 50/20/30. Après évaporation et séchage sous vide à 80°C, on obtient 3g de poudre blanche de [(pentaméthyl-1,1,2,3,3 indanyl-5) carbonyl]-6 naphtalène carboxylate de méthyle-2 de point de fusion: 139-141°C.

Le spectre $^1H$ R.M.N 60 MHz est conforme à la structure attendue.

| Analyse élémentaire: $C_{27}H_{28}O_3$ | | | | | | |
|---|---|---|---|---|---|---|
| Calculé:<br>Trouvé: | C: | 80,97<br>81,09 | H: | 7,05<br>7,14 | O: | 11,98<br>12,07 |

## EXEMPLE IV

Préparation de l'acide [(pentaméthyl-1,1,2,3,3 indanyl-5) carbonyl]-6 naphtalène carboxylique-2.

Composé de formule II dans laquelle: R' et R'' = oxo, $R_7$ = $-CH_3$ et R = $-CO_2H$.

Une suspension de 2g (5 mmoles) de [(pentaméthyl-1,1,2,3,3 indanyl-5) carbonyl]-6 naphtalène carboxylate de méthyle-2, obtenu à l'exemple III, est agitée 2h30 dans un mélange de 30cm$^3$ d'alcool et 30cm$^3$ de potasse aqueuse 6N chauffé au reflux. Après addition de 150cm$^3$ d'eau, l'alcool est éliminé par évaporation sous vide et la phase aqueuse est alors diluée à 500cm$^3$. Après refroidissement entre 0 et 5°C on acidifie par addition de 20cm$^3$ d'acide chlorhydrique 12N et le précipité obtenu est essoré, lavé à l'eau et séché sur potasse à 80-100°C. Après recristallisation dans un mélange hexane/acétone, on obtient 1,5g de cristaux blancs floconneux d'acide [(pentaméthyl-1,1,2,3,3 indanyl-5) carbonyl]-6 naphtalène carboxylique-2 de point de fusion: 237-8°C.

Le spectre $^1H$ R.M.N 250 MHz est conforme à la structure attendue.

| Analyse élémentaire: $C_{26}H_{26}O_3$ | | | | | | |
|---|---|---|---|---|---|---|
| Calculé:<br>Trouvé: | C: | 80,80<br>80,86 | H: | 6.78<br>6,77 | O: | 12,42<br>12,30 |

## EXEMPLE V

Préparation de l'acide [(pentaméthyl-1,1,2,3,3 indanyl-5) hydroxyméthyl]-6 naphtalène carboxylique-2.

Composé de formule II dans laquelle: R' = OH, R'' = H, $R_7$ = $-CH_3$ et R = $-CO_2H$.

A une solution de 0,7g (1,8 mmoles) d'acide [(pentaméthyl-1,1,2,3,3 indanyl-5) carbonyl]-6 naphtalène carboxylique-2, obtenu à l'exemple IV, dans 25cm$^3$ de tétrahydrofuranne anhydre, on ajoute, par portions en 15mn, 205mg (5,4 mmoles) de borohydrure de sodium et agite une nuit à température ambiante. La réduction une fois complète, le milieu réactionnel est refroidi entre 0 et 5°C, puis acidifié lentement par addition d'acide chlorhydrique 0,1N. Après extraction à l'éther éthylique, la phase éthérée est lavée à l'eau, séchée sur sulfate de sodium et évaporée à sec. Après recristallisation du produit brut obtenu dans un mélange hexane/acétone, on obtient 0,55g de cristaux blancs d'acide [(pentaméthyl-1,1,2,3,3 indanyl-5) hydroxyméthyl]-6 naphtalène carboxylique-2 de point de fusion: 215-7°C.

Le spectre $^1H$ R.M.N 250 MHz est conforme à la structure attendue.

| Analyse élémentaire: $C_{26}H_{28}O_3$ | | | | | | |
|---|---|---|---|---|---|---|
| Calculé:<br>Trouvé: | C: | 80,38<br>80,61 | H: | 7,26<br>7,32 | O: | 12,36<br>12,04 |

EXEMPLE VI

Préparation du [(tétraméthyl-5,5,8,3 tétrahydro-5,6,7,8 naphtyl-2) carbonyl]-6 naphtalène carboxylate de méthyle-2.

Composé de formule III dans laquelle $R_1 = R_2 = R_3 = R_4 = -CH_3$, R' et R'' = oxo et $R = -CO_2CH_3$.

A une suspension de 1,5g (11,2 mmoles) de chlorure d'aluminium anhydre dans 10cm$^3$ de dichlorométhane anhydre, on ajoute goutte à goutte une solution de 1,88g (10 mmoles) de tétrahydro-1,2,3,4 tétraméthyl-1,1,4,4 naphtalène et de 2,49g (10 mmoles) de chlorure de l'acide méthoxy carbonyl-6 naphtalène carboxylique-2 dans 60cm$^3$ de dichlorométhane anhydre. Le mélange est agité pendant 4 heures à température ambiante, puis versé dans 100cm$^3$ d'eau glacée acidulée. La phase organique est décantée. La phase aqueuse est extraite encore une fois à l'aide de 100cm$^3$ de dichlorométhane. Les phases de chlorure de méthylène sont rassemblées, lavées au bicarbonate de sodium, séchées sur sulfate de sodium puis concentrées. On obtient 3,9g d'un liquide jaune qui cristallise à température ordinaire. Ce solide est lavé au méthanol puis recristallisé dans 70cm$^3$ d'isopropanol.

On obtient 2,1g de [(tétraméthyl-5,5,8,8 tétrahydro-5,6,7,8 naphtyl-2) carbonyl]-6 naphtalène carboxylate de méthyle-2.

L'isopropanol est évaporé, le solide obtenu est lavé par le minimum de méthanol puis ensuite recristallisé dans ce solvant. On isole ainsi une seconde fraction de 0,7g de produit attendu identique à celui obtenu précédemment sous forme de cristaux blancs dont le point de fusion est de 134° C.

Le spectre R.M.N. $^1$H 60 MHz est conforme à la structure attendue.

| Analyse élémentaire: $C_{27}H_{28}O_3$ | | | | | | |
|---|---|---|---|---|---|---|
| Calculé:<br>Trouvé: | C: | 80,97<br>80,85 | H: | 7,05<br>7,00 | O: | 11,98<br>12,02 |

EXEMPLE VII

Préparation de l'acide [(tétraméthyl-5,5,8,8 tétrahydro-5,6,7,8 naphtyl-2) carbonyl]-6 naphtalène carboxylique-2

Composé de formule III dans laquelle: $R_1 = R_2 = R_3 = R_4 = -CH_3$, R' et R'' = oxo et $R = -CO_2H$.

Une suspension de 2,5g de [(tétraméthyl-5,5,8,8 tétrahydro-5,6,7,8 naphtyl-2) carbonyl]-6 naphtalène carboxylate de méthyle-2 obtenu à l'exemple VI est agitée pendant trois heures dans un mélange de 30cm$^3$ de méthanol et 30cm$^3$ de potasse aqueuse 6N à une température comprise entre 50° et 60° C puis abandonnée une nuit à température ordinaire. Après avoir ajouté 40cm$^3$ d'eau, le méthanol est éliminé par évaporation sous vide. La phase aqueuse ainsi obtenue est refroidie entre 0° et 5° C, puis acidifiée à pH≃1 par addition d'acide chlorhydrique 6N. Le précipité obtenu est alors essoré, lavé à l'eau, séché à 80° C sur potasse.

Après deux recristallisations, l'une dans 40cm$^3$ d'isopropanol, l'autre dans 100cm$^3$ de méthanol, 1,7g d'acide [(tétraméthyl-5,5,8,8 tétrahydro-5,6,7,8 naphtyl-2) carbonyl]-6 naphtalène carboxylique-2 est isolé sous forme de cristaux blancs dont le point de fusion est de 224° C.

Le spectre de R.M.N. $^1$H 250 MHz est conforme à la structure attendue.

| Analyse élémentaire: $C_{26}H_{26}O_3$ | | | | | | |
|---|---|---|---|---|---|---|
| Calculé:<br>Trouvé: | C: | 80,80<br>80,57 | H: | 6,78<br>6,93 | O: | 12,42<br>12,50 |

EXEMPLE VIII

Préparation du N-éthyl [(tétraméthyl-5,5,8,8 tétrahydro-5,6,7,8 naphtyl-2) carbonyl]-6 naphtalène carboxamide-2.

Composé de formule III dans laquelle: $R_1 = R_2 = R_3 = R_4 = -CH_3$, R' et R'' = oxo et R = $-CONHC_2H_5$.

A une suspension de 1g (2,5 mmoles) d'acide [(tétraméthyl-5,5,8,8 tétrahydro-5,6,7,8 naphtyl-2) carbonyl]-6 naphtalène carboxylique-6, obtenu à l'exemple VII, dans 10cm$^3$ de dichlorométhane anhydre, on ajoute 0,49g (3 mmoles) de N,N'-carbonyl diimidazole sous agitation à température ambiante.

Le gaz carbonique se dégage. Après une heure d'agitation 0,17cm$^3$ (2,6 mmoles) d'éthylamine anhydre sont ajoutés. Une heure après, l'acide de départ est totalement transformé en amide correspondant.

Le milieu réactionnel est alors dilué par 20cm$^3$ de dichlorométhane, lavé avec 20cm$^3$ de soude normale, puis à l'eau jusqu'à pH neutre des eaux de lavage, séché sur sulfate de sodium et concentré à sec.

On obtient 1g de solide beige que l'on recristallise dans un mélange éther isopropylique-acétonitrile. On isole ainsi, après avoir essoré et séché les cristaux, 0,65g de N-éthyl [(tétraméthyl-5,5,8,8 tétrahydro-5,6,7,8 naphtyl-2) carbonyl]-6 naphtalène carboxamide-2, sous forme de cristaux blancs dont le point de fusion est de 155° C.

Les spectres R.M.N$^1$H et infra-rouge correspondant à la structure attendue.

| Analyse élémentaire: $C_{28}H_{31}NO_2$ | | | | | | | | |
|---|---|---|---|---|---|---|---|---|
| Calculé:<br>Trouvé: | C: | 81,32<br>81,33 | H: | 7,56<br>7,53 | N: | 3,39<br>3,30 | O: | 7,74<br>7,72 |

EXEMPLE IX

Préparation de l'acide [(tétraméthyl-5,5,8,8 tétrahydro-5,6,7,8 naphtyl-2) hydroxyméthyl]-6 naphtalène carboxylique-2.

Composé de formule III dans laquelle: $R_1 = R_2 = R_3 = R_4 = -CH_3$, R' = OH, R'' = H et R = $-CO_2H$.

A une solution de 0,7g (1,8 mmoles) d'acide [(tétraméthyl-5,5,8,8 tétrahydro-5,6,7,8 naphtyl-2) carbonyl]-6 naphtalène carboxylique-2, obtenu selon l'exemple VII, dans 25cm$^3$ de tétrahydrofuranne anhydre, on ajoute par portions, à environ 10mn, 170mg (4,5 mmoles) de borohydrure de sodium et agite 20h à température ambiante. La réduction une fois complète, le milieu réactionnel est refroidi entre 0 et 5° C, puis acidifié par addition lente d'acide chlorhydrique 0,1N. Après extraction à l'éther éthylique, la phase éthérée est lavée à l'eau, séchée sur sulfate de sodium et évaporée à sec. Le solide obtenu est recristallisé dans un mélange toluène/hexane. Après séchage, on obtient 0,6g de cristaux blancs d'acide [-(tétraméthyl-5,5,8,8 tétrahydro-5,6,7,8 naphtyl-2) hydroxyméthyl]-6 naphtalène carboxylique-2 de point de fusion: 216-8° C.

Le spectre $^1$H R.M.N 250 MHz est conforme à la structure attendue.

| Analyse élémentaire: $C_{26}H_{28}O_3$ | | | | | | |
|---|---|---|---|---|---|---|
| Calculé:<br>Trouvé: | C: | 80,38<br>80,53 | H: | 7,26<br>7,28 | O: | 12,36<br>12,31 |

EXEMPLE X

Préparation du N-éthyl [(tétraméthyl-5,5,8,8 tétrahydro-5,6,7,8 naphtyl-2) hydroxyméthyl]-6 naphtalène carboxamide-2.

Composé de formule III dans laquelle: $R_1 = R_2 = R_3 = R_4 = -CH_3$, R' = OH, R'' = H et R = $-CONHC_2H_5$.

A une solution de 0,85g (2 mmoles) de N-éthyl [(tétraméthyl-5,5,8,8 tétrahydro-5,6,7,8 naphtyl-2) carbonyl]-6 naphtalène carboxamide-2 obtenu selon l'exemple VIII dans 25cm$^3$ de tétrahydrofuranne anhydre, on ajoute 300mg (8 mmoles) de borohydrure de sodium et agite 72h à température ambiante. Le

milieu réactionnel est alors refroidi entre 0 et 5° C, acidifié par addition lente d'acide chlorhydrique 0,1N puis extrait à l'éther éthylique. La phase éthérée est lavée à l'eau, séchée sur sulfate de sodium et évaporée à sec. Le solide blanc obtenu est recristallisé dans un mélange éther isopropylique/acétone. Après séchage, on obtient 0.7g d'aiguilles blanches de N-éthyl [(tétraméthyl-5,5,8,8 tétrahydro-5,6,7,8 naphtyl-2) hydroxyméthyl]-6 naphtalène carboxamide-2 de point de fusion: 175° C.

Le spectre $^1H$ R.M.N 80 MHz est conforme à la structure attendue.

| Analyse élémentaire: $C_{28}H_{33}NO_2$ | | | | | | | | |
|---|---|---|---|---|---|---|---|---|
| Calculé:<br>Trouvé: | C: | 80,92<br>81,00 | H: | 8,00<br>8,11 | N: | 3,37<br>3,53 | O: | 7,70<br>7,88 |

EXEMPLE XI

Préparation du (tétraméthyl-5,5,8,8 tétrahydro-5,6,7,8 naphtyl-2) -1 (carboxy-6 naphtyl-2)-1 méthane.

Composé de formule III dans laquelle: $R_1 = R_2 = R_3 = R_4 = -CH_3$, R' = R'' = H et $R = -CO_2H$.

A une suspension de 2g (0,03 mole) de zinc en poudre dans 20cm$^3$ d'acide acétique glacial, on ajoute 1g (2,6 mmoles) d'acide [(tétraméthyl-5,5,8,8 tétrahydro-5,6,7,8 naphtyl-2) carbonyl]-6 naphtalène carboxylique-2 obtenu selon l'exemple VII et chauffe 1h au reflux. On ajoute 2cm$^3$ d'acide chlorhydrique 12N et le reflux est maintenu 45mn. Après refroidissement à température ambiante et addition de 20cm$^3$ d'acide chlorhydrique 12N, le milieu réactionnel est dilué par 100cm$^3$ d'eau et extrait au dichlorométhane. La phase organique est lavée à l'eau, séchée sur sulfate de sodium et concentrée sous pression réduite. Le produit brut obtenu est recristallisé dans un mélange hexane/acétone. On obtient 0.7g de cristaux blancs de (tétraméthyl-5,5,8,8 tétrahydro-5,6,7,8 naphtyl-2) -1 (carboxy-6 naphtyl-2) -1 méthane de point de fusion: 212° C.

Le spectre $^1H$ R.M.N 80 MHz est conforme à la structure attendue.

| Analyse élémentaire: $C_{26}H_{28}O_2$ | | | | | | |
|---|---|---|---|---|---|---|
| Calculé:<br>Trouvé: | C: | 83,83<br>83,63 | H: | 7,58<br>7,63 | O: | 8,59<br>8,73 |

EXEMPLE XII

Préparation de l'acide [(tétraméthyl-5,5,8,8 tétrahydro-5,6,7,8 naphtyl-2) acétoxyméthyl]-6 naphtalène carboxylique-2.

Composé de formule III dans laquelle: $R_1 = R_2 = R_3 = R_4 = -CH_3$, $R' = -OCOCH_3$, R'' = H et $R = -CO_2H$.

Une solution de 0,5g (1,29 mmoles) d'acide [(tétraméthyl-5,5,8,8 tétrahydro-5,6,7,8 naphtyl-2) hydroxyméthyl]-6 naphtalène carboxylique-2 obtenu à l'exemple IX dans 50cm$^3$ de dichlorométhane anhydre et 3,5cm$^3$ (31 mmoles) d'anhydride acétique est agitée une semaine à température ambiante. On verse alors dans 50cm$^3$ d'eau et la phase de dichlorométhane est décantée, lavée abondamment à l'eau, séchée sur sulfate de sodium et concentrée sous pression réduite. L'huile obtenue est purifiée par chromatographie sur silice 60 dans le mélange éluant acide acétique/dioxanne/toluène 2/8/90. Après évaporation et séchage, le solide blanc obtenu est recristallisé dans de l'hexane contenant une trace d'acétone. On obtient 0,33g de cristaux blancs d'acide [(tétraméthyl-5,5,8,8 tétrahydro-5,6,7,8 naphtyl-2) acétoxyméthyl]-6 naphtalène carboxylique-2 de point de fusion: 178° C.

Le spectre $^1H$ R.M.N 80 MHz est conforme à la structure attendue.

| Analyse élémentaire: $C_{28}H_{30}O_4$ | | | | | | |
|---|---|---|---|---|---|---|
| Calculé:<br>Trouvé: | C: | 78,11<br>78,08 | H: | 7,02<br>7,07 | O: | 14,87<br>14,66 |

EXEMPLE XIII

Préparation du [(tétraméthyl-5,5,8,8 tétrahydro-5,6,7,8 naphtyl-2)-2 éthényl-2]-6 naphtalène carboxylate de méthyle-2.

Composé de formule III dans laquelle: $R_1 = R_2 = R_3 = R_4 = -CH_3$, R' et R'' = méthano et $R = -CO_2CH_3$.

A une solution de 20cm$^3$ de tétrahydrofuranne anhydre agitée à température ambiante, on ajoute 1,3g (3,1 mmoles) d'un mélange équimoléculaire de bromure de triphénylméthylphosphonium et d'amidure de sodium puis en 10mn, 1,2g (3 mmoles) de [(tétraméthyl-5,5,8,8 tétrahydro-5,6,7,8 naphtyl-2) carbonyl]-6 naphtalène carboxylate de méthyle-2, obtenu selon l'exemple VI. La réaction est complète après 6h d'agitation à température ambiante. Après addition de 100cm$^3$ de toluène puis de 50g de silice 60, le mélange réactionnel est filtré sur célite et le filtrat est concentré sous pression réduite. Le solide obtenu est purifié par chromatographie sur gel de silice 60 par élution au dichlorométhane suivie d'une recristallisation dans le méthanol. Après séchage, on obtient 0,9g d'aiguilles blanches de [(tétraméthyl-5,5,8,8 tétrahydro-5,6,7,8 naphtyl-2)-2 éthényl-2]-6 naphtalène carboxylate de méthyle-2 de point de fusion: 135 °C.

Le spectre $^1$H R.M.N 250 MHz est conforme à la structure attendue.

| Analyse élémentaire : $C_{28}H_{30}O_2$ | | | | | | |
|---|---|---|---|---|---|---|
| Calculé:<br>Trouvé: | C: | 84,38<br>84,28 | H: | 7,59<br>7,54 | O: | 8,03<br>8,12 |

EXEMPLE XIV

Préparation de l'acide [(tétraméthyl-5,5,8,8 tétrahydro-5,6,7,8 naphtyl-2)-2 éthényl-2]-6 naphtalène carboxylique-2.

Composé de formule III dans laquelle: $R_1 = R_2 = R_3 = R_4 = -CH_3$, R' et R'' = méthano et $R = -CO_2H$.

Une suspension de 0,75g de [(tétraméthyl-5,5,8,8 tétrahydro-5,6,7,8 naphtyl-2)-2 éthényl-2]-6 naphtalène carboxylate de méthyle-2, obtenu à l'exemple XIII, est agitée 1h dans un mélange de 15cm$^3$ d'alcool éthylique et 15cm$^3$ de potasse aqueuse 6N chauffé au reflux. Après addition de 100cm$^3$ d'eau, l'alcool est éliminé par évaporation sous vide. On récupère le carboxylate insoluble et on le reprend par un mélange de 100cm$^3$ de tétrahydrofuranne et de 50cm$^3$ d'eau. Après refroidissement entre 0 et 5 °C, on acidifie par addition d'acide chlorhydrique N puis extrait à l'éther. La phase organique est lavée à l'eau, séchée sur sulfate de sodium puis concentrée sous pression réduite. Le solide blanc obtenu est recristallisé dans un mélange heptane/acétone. Après séchage, on obtient 0,6g d'aiguilles blanches d'acide [(tétraméthyl-5,5,8,8 tétrahydro-5,6,7,8 naphtyl-2)-2 éthényl-2]-6 naphtalène carboxylique-2 de point de fusion: 220 °C.

Le spectre $^1$H R.M.N 250 MHz est conforme à la structure attendue.

| Analyse élémentaire: $C_{27}H_{28}O_2$ | | | | | | |
|---|---|---|---|---|---|---|
| Calculé:<br>Trouvé: | C: | 84,34<br>84,20 | H: | 7,34<br>7,30 | O: | 8,32<br>8,40 |

EXEMPLES XV et XVI

Préparation du [(tétraméthyl-5,5,8,8 tétrahydro-5,6,7,8 naphtyl-2) hydroxyméthyl]-6 naphtalène carbinol-2

Composé de formule III dans laquelle: $R_1 = R_2 = R_3 = R_4 = -CH_3$, R' = OH, R'' = H et $R = -CH_2OH$.

et du [(tétraméthyl-5,5,8,8 tétrahydro-5,6,7,8 naphtyl-2) butoxyméthyl]-6 naphtalène carbinol-2.

Composé de formule III dans laquelle: $R_1 = R_2 = R_3 = R_4 = -CH_3$, $R' = -OC_4H_9$ R'' = H et $R = -CH_2OH$.

A une solution de 1,55g (4 mmoles) d'acide [(tétraméthyl-5,5,8,8 tétrahydro-5,6,7,8 naphtyl-2) carbonyl]-6 naphtalène carboxylique-2, obtenu selon l'exemple VII, dans 5cm$^3$ de tétrahydrofuranne anhydre, refroidie

à -30° C et maintenue sous argon, on ajoute 40cm$^3$ de solution molaire de borane dans le tétrahydrofuranne et agite 2h en laissant revenir à température ambiante. La solution est abandonnée une nuit à température ambiante puis refroidie à environ 0° C, acidifiée par addition lente de 60cm$^3$ d'acide chlorhydrique N et extraite à l'éther éthylique. La phase organique est lavée à l'eau, séchée sur sulfate de sodium puis concentrée sous pression réduite, L'huile brute obtenue contient deux produits qui sont séparés par chromatographie sur gel de silice 60 dans le mélange éluant acide acétique/dioxanne/toluène 2/8/90. Chacun des produits est à nouveau purifié séparément par chromatographie sur silice dans le même mélange éluant. On obtient ainsi 0,6g de [(tétraméthyl-5,5,8,8 tétrahydro-5,6,7,8 naphtyl-2) hydroxyméthyl]-6 naphtalène carbinol-2 sous forme d'une poudre blanche de point de fusion: 157° C et 0.8g d'huile épaisse incolore correspondant au [(tétraméthyl-5,5,8,8 tétrahydro-5,6,7,8 naphtyl-2) butoxyméthyl]-6 naphtalène carbinol-2.

Les spectres $^1$H R.M.N 250 MHz des deux composés sont conformes aux structures décrites.

Analyses élémentaires respectives:

1) $C_{26}H_{30}O_2$

| | | | | | | |
|---|---|---|---|---|---|---|
| Calculé: | C: | 83,38 | H: | 8,07 | O: | 8,55 |
| Trouvé: | | 83,43 | | 8,08 | | 8,59 |

2) $C_{28}H_{34}O_2$

| | | | | | | |
|---|---|---|---|---|---|---|
| Calculé: | C: | 83,54 | H: | 8,51 | O: | 7,95 |
| Trouvé: | | 83,54 | | 8,72 | | 8,18 |

EXEMPLE XVII

Préparation de l'acide [(tétraméthyl-5,5,8,8 tétrahydro-5,6,7,8 naphtyl-2) butoxyméthyl]-6 naphtalène carboxylique-2.

Composé de formule III dans laquelle: $R_1 = R_2 = R_3 = R_4 = -CH_3$, $R' = -OC_4H_9$, $R'' = H$ et $R = -CO_2H$.

1ère méthode:

A une solution de 0,6g (1,5 mmoles) d'acide [(tétraméthyl-5,5,8,8 tétrahydro-5,6,7,8 naphtyl-2) hydrométhyl]-6 naphtalène carboxylique-2, obtenu à l'exemple IX, dans 20cm$^3$ de tétrahydrofuranne, agitée à température ambiante, on ajoute 10cm$^3$ d'acide chlorhydrique 12N puis 2g de chlorure de calcium anhydre.

Après 1h d'agitation, on ajoute 75cm$^3$ d'éther éthylique et décante la phase aqueuse. La phase organique est lavée à l'eau, séchée sur sulfate de sodium et évaporée à sec. On obtient ainsi 0,62g d'acide [(tétraméthyl-5,5,8,8 tétrahydro-5,6,7,8 naphtyl-2) chlorométhyl]-6 naphtalène carboxylique-2 brut (solide blanc). On ajoute alors 20cm$^3$ d'alcool butylique primaire, agite 30mn à 60° C puis concentre sous pression réduite. L'huile obtenue est purifiée par chromatographie sur gel de silice 60 dans le mélange éluant acide acétique/dioxanne/toluène 2/8/90. Après évaporation et séchage sous vide à 80° C, on obtient 0,54g d'acide [(tétraméthyl-5,5,8,8 tétrahydro-5,6,7,8 naphtyl-2) butoxyméthyl]-6 naphtalène carboxylique-2 sous la forme d'une poudre blanche de point de fusion: 71-73° C.

Le spectre $^1$H R.M.N 80 MHz est conforme à la structure attendue.

| Analyse élémentaire: $C_{30}H_{36}O_3$ | | | | | | |
|---|---|---|---|---|---|---|
| Calculé:<br>Trouvé: | C: | 81,04<br>80,64 | H: | 8,16<br>8,21 | O: | 10,80<br>11,20 |

2ème méthode :

A une solution de 0,4g (1 mmole) de [(tétraméthyl-5,5,8,8 tétrahydro-5,6,7,8 naphtyl-2) butoxyméthyl]-6

naphtalène carbinol-2, obtenu selon l'exemple XVI, dans $10cm^3$ d'acétone, agitée à température ambiante, on ajoute une solution de 0,3g d'anhydride chromique dans $1,5cm^3$ d'eau et $0,25cm^3$ d'acide sulfurique à 98%.

Après 5h d'agitation à température ambiante, le milieu réactionnel est dilué par $25cm^3$ d'eau et extrait 3 fois par $100cm^3$ d'éther éthylique. La phase éthérée est lavée à l'eau, séchée sur sulfate de sodium et évaporée à sec. Le solide brut obtenu est purifié par chromatographie sur gel de silice 60 dans le mélange éluant acide acétique/dioxanne/toluène 2/8/90. Après évaporation et séchage, on obtient 0,26g d'acide [-(tétraméthyl-5,5,8,8 tétrahydro-5,6,7,8 naphtyl-2) butoxyméthyl]-6 naphtalène carboxylique-2 sous la forme d'un solide blanc de point de fusion: 72-73° C.

Le spectre $^1H$ R.M.N 80 MHz est conforme à la structure attendue.

## EXEMPLE XVIII

### Préparation du [(tétraméthyl-5,5,8,8 tétrahydro-5,6,7,8 naphtyl-2) hydroxyméthyl]-6 naphtaldéhyde-2.

Composé de formule III dans laquelle: $R_1 = R_2 = R_3 = R_4 = -CH_3$, R' = OH, R'' = H et R = -CH = O.

A une solution de 1,4g (3,7 mmoles) de [(tétraméthyl-5,5,8,8 tétrahydro-5,6,7,8 naphtyl-2) hydroxyméthyl]-6 naphtalène carbinol-2 obtenu selon l'exemple XV dans $25cm^3$ de dichlorométhane anhydre, on ajoute par portions en 10mn, 0,8g (3,7 mmoles) de chlorochromate de pyridinium et agite 1h à température ambiante. Le milieu réactionnel est alors filtré sur célite et le filtrat est concentré sous pression réduite. Le produit brut obtenu est purifié par chromatographie sur gel de silice 60 dans le dichlorométhane suivie d'une recristallisation dans de l'hexane contenant un peu d'acétone.

Après filtration et séchage, on obtient 0,7g d'aiguilles blanches de [(tétraméthyl-5,5,8,8 tétrahydro-5,6,7,8 naphtyl-2) hydroxyméthyl]-6 naphtaldéhyde-2 de point de fusion: 148° C.

Le spectre $^1H$ R.M.N 250 MHz est conforme à la structure attendue.

| Analyse élémentaire: $C_{26}H_{28}O_2$ | | | | | | |
|---|---|---|---|---|---|---|
| Calculé:<br>Trouvé: | C: | 83,83<br>84,17 | H: | 7,58<br>7,37 | O: | 8,59<br>8,43 |

## EXEMPLE XIX

### Préparation du [(tétraméthyl-5,5,8,8 tétrahydro-5,6,7,8 naphtyl-2) hydroxyiminométhyl]-6 naphtalène carboxylate d'éthyle-2

Composé de formule III dans laquelle $R_1 = R_2 = R_3 = R_4 = -CH_3$, R' et R'' = hydroxyimino et R = $-CO_2C_2H_5$

Une suspension de 1,94g (5mmoles) d'acide [(tétraméthyl-5,5,8,8 tétrahydro-5,6,7,8 naphtyl-2) carbonyl]-6 naphtalène carboxylique-2 dans $100cm^3$ d'éthanol absolu contenant $0,2cm^3$ d'acide sulfurique à 98% est chauffée au reflux jusqu'à transformation complète en ester éthylique (16h).

On ajoute alors 695mg (10mmoles) de chlorhydrate d'hydroxylamine puis $1,9cm^3$ (13,5mmoles) de triéthylamine et chauffe à nouveau 15h au reflux. La solution est alors refroidie à température ambiante puis concentrée sous pression réduite. Le résidu est repris par un mélange tétrahydrofuranne/éther éthylique 50/50, lavé à l'acide chlorhydrique 0,1N puis à l'eau. La solution obtenue est séchée sur sulfate de sodium puis évaporée à sec. Le solide blanc ainsi isolé est purifié rapidement par chromatographie sur gel de silice 60 dans le dichlorométhane suivie d'une recristallisation dans le minimum d'éthanol bouillant. On obtient ainsi, après séchage, 1,9g d'aiguilles blanches de [(tétraméthyl-5,5,8,8 tétrahydro-5,6,7,8 naphtyl-2) hydroxyiminométhyl]-6 naphtalène carboxylate d'éthyle-2 de point de fusion : 179-180° C.

Le spectre $^1H$ RMN 80 MHz est conforme à la struture attendue.

| Analyse élémentaire : $C_{28}H_{31}NO_3$ | | | | | | | | |
|---|---|---|---|---|---|---|---|---|
| Calculé :<br>Trouvé : | C : | 78,29<br>78,31 | H : | 7,27<br>7,23 | N : | 3,26<br>3,39 | O : | 11,17<br>11,41 |

EXEMPLE XX

Préparation de l'acide [(tétraméthyl-5,5,8,8 tétrahydro-5,6,7,8 naphtyl-2) hydroxyiminométhyl]-6 naphtalène carboxylique-2

Composé de formule III dans laquelle $R_1 = R_2 = R_3 = R_4 = -CH_3$, R' et R'' = hydroxyimino et R = -COOH

Une solution de 0,76g (1,8mmoles) de [(tétraméthyl-5,5,8,8 tétrahydro-5,6,7,8 naphtyl-2) hydroxyiminométhyl]-6 naphtalène carboxylate d'éthyle-2, obtenu selon l'exemple XIX, dans 10cm$^3$ d'alcool absolu et 10cm$^3$ de potasse aqueuse 2N est chauffée 2h à 50-60°C. Après addition de 100cm3 d'eau, l'alcool est évaporé sous pression réduite. La solution aqueuse obtenue est refroidie entre 0 et 5°C puis acidifiée par 5cm$^3$ d'acide chlorhydrique 12N. Le précipité obtenu est essoré, lavé à l'eau, séché sous vide sur potasse à 70-80°C puis recristallisé dans un mélange hexane/acétone. Après séchage, on obtient 0,65g de cristaux blancs d'acide [(tétraméthyl-5,5,8,8 tétrahydro-5,6,7,8 naphtyl-2) hydroxyiminométhyl]-6 naphtalène carboxylique-2 de point de fusion : 247°C.

Les spectres RMN$^1$H 250 MHz et I.R. sont conformes à la structure attendue.

EXEMPLE XXI

Préparation du N-éthyl [(tétraméthyl-5,5,8,8 tétrahydro-5,6,7,8 naphtyl-2) hydroxyiminométhyl]-6 naphtalène carboxamide-2 :

Composé de formule III dans laquelle $R_1 = R_2 = R_3 = R_4 = -CH_3$, R' et R'' = hydroxyimino et R = $-CONHC_2H_5$.

A une suspension de 1,24g (3mmoles) de N-éthyl [(tétraméthyl-5,5,8,8 tétrahydro-5,6,7,8 naphtyl-2) carbonyl]-6 naphtalène carboxamide-2 et de 695mg (10mmoles) de chlorhydrate d'hydroxylamine dans 40cm$^3$ d'alcool absolu, on ajoute 1,06g (10mmoles) de carbonate de sodium et chauffe 24h au reflux. La solution est alors évaporée à sec et le résidu repris par 100cm$^3$ de dichlorométhane. Après lavage à l'eau la phase dichlorométhane est séchée sur sulfate de sodium et concentrée sous pression réduite. Le solide jaune pâle obtenu est purifié par chromatographie sur gel de silice 60 éluée avec un mélange toluène/dichlorométhane/acétate d'éthyle 5/3/2 suivie d'une recristallisation dans le toluène. On obtient ainsi 0,9g de cristaux blancs de N-éthyl [(tétraméthyl-5,5,8,8 tétrahydro-5,6,7,8 naphtyl-2) hydroxyiminométhyl]-6 naphtalène carboxamide-2 de point de fusion : 189°C.

Les spectres RMN$^1$H 250 MHz et I.R. sont conformes à la structure attendue.

| Analyse élémentaire : $C_{28}H_{32}N_2O_2$ | | | | | | | | |
|---|---|---|---|---|---|---|---|---|
| Calculé :<br>Trouvé : | C : | 78,47<br>77,99 | H : | 7,53<br>7,56 | N : | 6,54<br>6,53 | O : | 7,47<br>7,85 |

EXEMPLE XXII

Préparation du [(tétraméthyl-5,5,8,8 tétrahydro-5,6,7,8 naphtyl-2) aminométhyl]-6 naphtalène carboxylate d'éthyle-2 :

Composé de formule III dans laquelle $R_1 = R_2 = R_3 = R_4 = -CH_3$, R' = $-NH_2$, R'' = H et R = $-CO_2C_2H_5$

A une suspension de 1,5g (0,023 mole) de zinc en poudre dans 20cm$^3$ d'acide acétique glacial, on ajoute 0,9g (2,1 mmoles) de [(tétraméthyl-5,5,8,8tétrahydro-5,6,7,8 naphtyl-2) hydroxyiminométhyl]-6 naphtalène carboxylate d'éthyle-2 obtenu à l'exemple XIX et chauffe 30mn à 80°C. Après refroidissement à température ambiante, on ajoute 100cm$^3$ de dichlorométhane et filtre. Le filtrat est lavé trois fois par 50cm$^3$ de solution aqueuse d'ammoniaque à 5% puis à l'eau, séché sur sulfate de sodium et évaporé à sec. Le solide blanc obtenu est recristallisé dans l'hexane. Après séchage, on obtient 0,69g d'aiguilles blanches de [(tétraméthyl-5,5,8,8 tétrahydro-5,6,7,8 naphtyl-2) aminométhyl]-6 naphtalène carboxylate d'éthyle-2 de point de fusion : 126°C.

Le spectre RMN$^1$H 80 MHz est conforme à la structure attendue.

| Analyse élémentaire : $C_{28}H_{33}NO_2$ | | | | | | | | |
|---|---|---|---|---|---|---|---|---|
| Calculé :<br>Trouvé : | C : | 80,92<br>80,74 | H : | 8,00<br>8,03 | N : | 3,37<br>3,42 | O : | 7,70<br>7,97 |

EXEMPLE XXIII

Préparation de l'acide [(tétraméthyl-5,5,8,8 tétrahydro-5,6,7,8 naphtyl-2) aminométhyl]-6 naphtalène carboxylique-2

Composé de formule III dans laquelle $R_1 = R_2 = R_3 = R_4 = -CH_3$, R' = $NH_2$, R'' = H et R = $-CO_2H$

Une solution de 0,63g (1,51 mmoles) de [(tétraméthyl-5,5,8,8 tétrahydro-5,6,7,8 naphtyl-2) aminométhyl]-6 naphtalène carboxylate d'éthyle-2 obtenu à l'exemple XXII dans $10cm^3$ d'alcool absolu et $10cm^3$ de potasse aqueuse 2N est chauffée 2h à 60-70°C. Après addition de $100cm^3$ d'eau, l'alcool est évaporé sous pression réduite. La solution aqueuse obtenue est refroidie entre 0 et 5°C puis acidifiée par addition d'acide chlorhydrique 12N. Le précipité obtenu est essoré, lavé à l'eau, séché sous vide à 80°C sur potasse puis lavé à l'éther éthylique. Après séchage, on obtient 0,54g d'acide [(tétraméthyl-5,5,8,8 tétrahydro-5,6,7,8 naphtyl-2) aminométhyl]-6 naphtalène carboxylique-2 sous la forme d'une poudre blanche de point de fusion : 286-288°C.

Les spectres RMN$^1$H 250 MHz et I.R. sont conformes à la structure attendue.

EXEMPLE XXIV

Préparation du [(méthano-5,8 tétrahydro-5,6,7,8 naphtyl-2) carbonyl]-6 naphtalène carboxylate de méthyle-2.

Composé de formule IV dans laquelle R' et R'' = oxo et R = $-CO_2CH_3$.

Une suspension de 1,08g (7,5 mmoles) de méthano-1,4 tétrahydro-1,2,3,4 naphtalène et de 2g (8 mmoles) de chlorure de l'acide méthoxycarbonyl-6 naphtalène carboxylique-2 dans $30cm^3$ de dichloro-1,2 éthane anhydre, on ajoute par portions en 45mn, 1,3g (9,75 mmoles) de chlorure d'aluminium anhydre. Le mélange est agité pendant 5 heures à température ambiante puis versé dans $80cm^3$ d'eau glacée acidulée. La phase organique est décantée. La phase aqueuse est extraite encore une fois à l'aide de $60cm^3$ de dichloro-1,2 éthane. Les phases de dichloro-1,2 éthane sont rassemblées lavées au bicarbonate de sodium, séchées sur sulfate de sodium puis concentrées. Le solide obtenu est séché sous vide à 60°C puis recristallisé d'abord dans l'acétate d'éthyle puis dans l'isopropanol. On obtient ainsi 1g de [(méthano-5,8 tétrahydro-5,6,7,8 naphtyl-2) carbonyl]-6 naphtalène carboxylate de méthyle-2 sous forme de cristaux blancs dont le point de fusion est de 119°C.

Le spectre RMN$^1$H 60MHz est conforme à la structure attendue.

| Analyse élémentaire: $C_{24}H_{20}O_3$ | | | | | | |
|---|---|---|---|---|---|---|
| Calculé:<br>Trouvé: | C: | 80,87<br>80,82 | H: | 5,66<br>5,69 | O: | 13,47<br>13,25 |

EXEMPLE XXV

Préparation de l'acide [(méthano-5,8 tétrahydro-5,6,7,8 naphtyl-2) carbonyl]-6 naphtalène carboxylique-2.

Composé de formule IV dans laquelle: R' et R'' = oxo et R = $-CO_2H$.

Une suspension de 0,88g (2,46 mmoles) de [(méthano-5,8 tétrahydro-5,6,7,8 naphtyl-2) carbonyl]-6 naphtalène carboxylate de méthyle-2 obtenu à l'exemple XXIV est agitée pendant deux heures dans un mélange de $15cm^3$ d'alcool et $15cm^3$ de potasse aqueuse 6N chauffé au reflux. Après avoir ajouté $60cm^3$ d'eau, l'alcool est éliminé par évaporation sous vide. La phase aqueuse ainsi obtenue est refroidie entre 0 et 5°C puis acidifiée par addition de $15cm^3$ d'acide chlorhydrique 12N. Le précipité obtenu est essoré, lavé à l'eau, séché à 80°C sur potasse.

Après recristallisation dans $110cm^3$ de méthanol, on obtient 0,62g de cristaux blancs d'acide [(méthano-5,8 tétrahydro-5,6,7,8 naphtyl-2) carbonyl]-6 naphtalène carboxylique-2 dont le point de fusion est de 243 ° C.

Les spectres de R.M.N. $^1H$ 250MHz et $^{13}C$ sont conformes à la structure attendue.

| Analyse élémentaire: $C_{23}H_{18}O_3$ | | | | | | |
|---|---|---|---|---|---|---|
| Calculé:<br>Trouvé: | C: | 80,68<br>80,83 | H: | 5,30<br>5,43 | O: | 14,02<br>13,71 |

EXEMPLE XXVI

Préparation du [(tétrahydro-5,6,7,8 naphtyl-2) carbonyl]-6 naphtalène carboxylate de méthyle-2.

Composé de formule III dans laquelle: $R_1 = R_2 = R_3 = R_4 = H$ , R' et R'' = oxo et $R = -CO_2CH_3$.

A une suspension de 1,59g (12 mmoles) de tétrahydro-1,2,3,4 naphtalène et de 3g (12,05 mmoles) de chlorure de l'acide méthoxycarbonyl-6 naphtalène carboxylique-2 dans 60cm$^3$ de dichloro-1,2 éthane anhydre, on ajoute par portions, 2,4g (18 mmoles) de chlorure d'aluminium anhydre. Le mélange est agité pendant 6 heures à température ambiante puis versé dans 100cm$^3$ d'eau glacée acidulée. La phase organique est décantée. La phase aqueuse est extraite encore une fois à l'aide de 100cm$^3$ de dichloro-1,2 éthane. Les phases de dichloroéthane sont rassemblées, lavées au bicarbonate de sodium, séchées sur sulfate de sodium puis concentrées à sec. L'ester brut obtenu est purifié rapidement par chromatographie sur gel de silice avec un mélange hexane/éther 8/2, puis recristallisé dans l'isopropanol. On obtient ainsi 2g de cristaux blancs de [(tétrahydro-5,6,7,8 naphtyl-2) carbonyl]-6 naphtalène carboxylate de méthyle-2 dont le point de fusion est de 171 ° C.

Le spectre de R.M.N. $^1H$ 60MHz est conforme à la structure attendue.

| Analyse élémentaire: $C_{23}H_{20}O_3$ | | | | | | |
|---|---|---|---|---|---|---|
| Calculé:<br>Trouvé: | C: | 80,21<br>80,16 | H: | 5,85<br>5,91 | O: | 13,94<br>13,85 |

EXEMPLE XXVII

Préparation de l'acide [(tétrahydro-5,6,7,8 naphtyl-2) carbonyl]-6 naphtalène carboxylique-2.

Composé de formule III avec $R_1 = R_2 = R_3 = R_4 = H$, R' et R'' = oxo et R = -COOH.

Une suspension de 1,50g (4,35 mmoles) de [(tétrahydro-5,6,7,8 naphtyl -2) carbonyl]-6 naphtalène carboxylate de méthyle-2 obtenu à l'exemple XXVI est agitée pendant trois heures dans un mélange de 25cm$^3$ d'alcool et de 25cm$^3$ de potasse aqueuse 6N chauffé au reflux. Après avoir ajouté 50cm$^3$ d'eau, l'alcool est éliminé par évaporation sous vide. La phase aqueuse ainsi obtenue est diluée à 500cm$^3$ pour solubiliser entièrement le carboxylate puis acidifiée par addition de 25cm$^3$ d'acide chlorhydrique 12N. Le précipité obtenu est essoré, lavé à l'eau, séché à 80 ° C sur potasse.

Après recristallisation d'abord dans l'isopropanol puis dans un mélange méthanol/acétone 8/2 on obtient 1,1g de cristaux blancs d'acide [(tétrahydro-5,6,7,8 naphtyl-2) carbonyl]-6 naphtalène carboxylique-2 dont le point de fusion est de 267 ° C.

Les spectres de R.M.N. $^1H$ 250MHz et $^{13}C$ sont conformes à la structure attendue.

| Analyse élémentaire: $C_{22}H_{18}O_3$ | | | | | | |
|---|---|---|---|---|---|---|
| Calculé:<br>Trouvé: | C: | 79,98<br>80,18 | H: | 5,49<br>5,52 | O: | 14,53<br>14,24 |

EXEMPLE XXVIII

Préparation du N-éthyl [(tétrahydro-5,6,7,8 naphtyl-2) carbonyl]-6 naphtalène carboxamide-2.

Composé de formule III avec $R_1 = R_2 = R_3 = R_4 = H$, R' et R'' = oxo et R = -CONH $C_2H_5$.

Une suspension de 165g (0,5 mmole) d'acide [(tétrahydro-5,6,7,8 naphtyl-2) carbonyl]-6 naphtalène carboxylique-2, obtenu à l'exemple XXVII et de 97,5mg (0,6 mmole) de N,N'-carbonyldiimidazole dans 5cm$^3$ de dichlorométhane sec est agitée 2 heures à température ambiante. On ajoute alors 0,04cm$^3$ (0,6 mmole) d'éthylamine anhydre à la solution obtenue. Après 30 minutes d'agitation, le milieu réactionnel est dilué par 10cm$^3$ d'eau, lavé successivement par 10cm$^3$ de soude normale, 2 fois 10cm$^3$ d'eau, 10cm$^3$ d'acide chlorhydrique N, et 10cm$^3$ d'eau. La phase dichlorométhane est séchée sur sulfate de sodium puis évaporée à sec. L'amide brut est séché sous vide à 60 °C puis recristallisé dans de l'éther isopropylique contenant un peu de méthanol. On obtient 130mg d'aiguilles jaune pâle de N-éthyl [(tétrahydro-5,6,7,8 naphtyl-2) carbonyl]-6 naphtalène carboxamide-2 dont le point de fusion est de 137 °C.

Les spectres R.M.N. $^1$H 250MHz et $^{13}$C sont conformes à la structure attendue.

| Analyse élémentaire: $C_{24}H_{23}NO_2$ | | | | | | | | |
|---|---|---|---|---|---|---|---|---|
| Calculé: | C: | 80,64 | H: | 6,49 | N: | 3,92 | O: | 8,95 |
| Trouvé: | | 80,49 | | 6,55 | | 3,86 | | 8,75 |

EXEMPLE XXIX

Préparation du [(tétraméthyl-5,5,8,8 tétrahydro-5,6,7,8 naphtyl-2) carbonyl]-6 naphtalène carboxaldéhyde-2.

Composé de formule III avec $R_1 = R_2 = R_3 = R_4 = -CH_3$ , R' et R'' = oxo et R = -CH = O.

Ce composé est préparé en deux étapes à partir de l'acide [(tétraméthyl-5,5,8,8 tétrahydro-5,6,7,8 naphtyl-2)-6 carbonyl]-6 naphtalène carboxylique-2 préparé à l'exemple VII. Dans une première étape les fonctions carbonyle et acide carboxylique sont réduites en alcool. Dans une deuxième étape le [-(tétraméthyl-5,5,8,8 tétrahydro-5,6,7,8 naphtyl-2) hydroxyméthyl]-6 naphtalène carbinol-2 est oxydé en aldéhyde attendu.

a) Préparation du [(tétraméthyl-5,5,8,8 tétrahydro-5,6,7,8 naphtyl-2) hydroxyméthyl]-6 naphtalène carbinol-2.

A une suspension de 225mg (6 mmoles) d'hydrure de lithium aluminium dans 10cm$^3$ de tétrahydrofuranne anhydre agitée à 0 °C, on ajoute en une fois 1,16g (3 mmoles) d'acide préparé suivant l'exemple II. Après une heure, le milieu est agité à température ordinaire, la réaction étant suivie en C.C.M. Lorsque la totalité du produit de départ est transformée on ajoute lentement 100cm$^3$ d'acide chlorhydrique 0,1N puis le mélange est extrait 4 fois par 25cm$^3$ d'éther éthylique. Les phases éthérées sont décantées, séchées sur sulfate de sodium et le solvant est éliminé par évaporation sous vide.

Le diol brut obtenu est purifié par passage sur colonne de gel de silice et élué au mélange acide acétique-dioxanne-toluène (2-8-90).

Après évaporation de l'éluant on isole 0,9g de [(tétraméthyl-5,5,8,8, tétrahydro-5,6,7,8 naphtyl-2) hydroxyméthyl]-6 naphtalène carbinol-2 sous forme de cristaux blancs dont le point de fusion est de 157 °C.

Le spectre R.M.N. $^1$H 60MHz est conforme à la structure attendue.

b) Oxydation du diol précédent

A une solution du diol préparé ci-dessus de 0,8g (2,1 mmoles) dans 20cm$^3$ de dichlorométhane anhydre, agitée à la température ordinaire, on ajoute 0,55g (2,5 mmoles) de chlorochromate de pyridinium. Après 45mn on ajoute 30g de gel de silice et l'ensemble est filtré sur célite. Le filtre est lavé au dichlorométhane et la solution est concentrée sous pression réduite. Le produit obtenu est purifié par chromatographie sur gel de silice et élué au mélange acide acétique-dioxanne-toluène (2/8/90). Après concentration des phases d'élution, puis recristallisation dans l'hexane on obtient 0,52g de [(tétraméthyl-5,5,8,8 tétrahydro-5,6,7,8 naphtyl-2) carbonyl]-6 naphtalène carboxaldéhyde-2 sous forme de cristaux blancs dont le point de fusion est de 131 °C.

Le spectre R.M.N. $^1$H 250MHz est conforme à la structure attendue.

| Analyse élémentaire: $C_{26}H_{26}O_2$ | | | | | | |
|---|---|---|---|---|---|---|
| Calculé:<br>Trouvé: | C: | 84,29<br>84,21 | H: | 7,07<br>6,96 | O: | 8,64<br>8,46 |

## EXEMPLE XXX

Préparation du [(tétraméthyl-5,5,8,8 tétrahydro-5,6,7,8 naphtyl-2) carbonyl]-6 naphtalène carbinol-2.

Composé de formule III avec $R_1 = R_2 = R_3 = R_4 = -CH_3$ , R' et R'' = oxo et $R = -CH_2OH$.

A une solution de 0,3g de potasse à 85% dans 10cm³ de méthanol, agitée à 60° C, on ajoute 0,6g (1,6 mmoles) de [(tétraméthyl-5,5,8,8 tétrahydro-5,6,7,8 naphtyl-2) carbonyl]-6 formyl-2 naphtalène et 0,16cm³ (1,9 mmoles) de formaldéhyde en solution aqueuse à 37%. Après 2h d'agitation à 60-70° C, le méthanol est distillé sous pression normale. On ajoute alors 10cm³ d'eau au résidu chaud et refroidit à température ambiante. Le précipité formé est extrait par quatre fois 25cm³ d'éther éthylique. Les phases éthérées sont lavées à l'eau puis séchées sur sulfate de sodium et évaporées à sec sous pression réduite. Le produit brut est purifié par chromatographie sur gel de silice 60 dans le mélange éluant acide acétique/dioxanne/toluène 2/8/90 suivie d'une recristallisation dans un mélange hexane/acétone. Après séchage, on obtient 240mg de cristaux blancs de [(tétraméthyl-5,5,8,8 tétrahydro-5,6,7,8 naphtyl-2) carbonyl]-6 naphtalène carbinol-2 dont le point de fusion est de 117° C.

Le spectre R.M.N. $^1H$ 250 MHz correspond à la structure attendue.

| Analyse élémentaire: $C_{26}H_{28}O_2$ | | | | | | |
|---|---|---|---|---|---|---|
| Calculé:<br>Trouvé: | C: | 83,83<br>83,77 | H: | 7,58<br>7,55 | O: | 8,59<br>8,75 |

## EXEMPLE XXXI

Préparation du N-hydroxy-2' éthoxy-2 éthyl [(tétraméthyl-5,5,8,8 tétrahydro-5,6,7,8 naphtyl-2) carbonyl]-6 naphtalène carboxamide-2.

Composé de formule III avec $R_1 = R_2 = R_3 = R_4 = -CH_3$ , R' et R'' = oxo et $R = -CONH(CH_2)_2O-(CH_2)_2-OH$.

A une suspension de 0,9g (2,33 mmoles) d'acide [(tétraméthyl-5,5,8,8 tétrahydro-5,6,7,8 naphtyl-2) carbonyl]-6 naphtalène carboxylique-2 dans 9cm³ de dichlorométhane anhydre, on ajoute 490mg (3,03 mmoles) de N,N'-carboxyldiimidazole. Après 1h d'agitation à température ambiante, on ajoute 0,27cm³ (2,57 mmoles) de N-hydroxyéthoxy-2 éthylamine à la solution obtenue. Le milieu réactionnel est agité 3h à température ambiante puis dilué par 25cm³ de dichlorométhane et lavé à l'eau et à l'acide chlorhydrique dilué. La phase dichlorométhane est séchée sur sulfate de sodium puis évaporée à sec. L'amide brut est purifié par chromatographie sur gel de silice 60 dans le mélange éluant dichlorométhane/tétrahydrofuranne 70/30. Après évaporation et séchage, on obtient 0,65g de cristaux blancs de N-hydroxy-2' éthoxy-2 éthyl [-(tétraméthyl-5,5,8,8 tétrahydro-5,6,7,8 naphtyl-2) carbonyl]-6 naphtalène carboxamide-2 devenant vitreux à 80° C.

Le spectre R.M.N. $^1H$ 250MHz est conforme à la structure attendue.

| Analyse élémentaire: $C_{30}H_{35}NO_4$ | | | | | | | | |
|---|---|---|---|---|---|---|---|---|
| Calculé:<br>Trouvé: | C: | 76,08<br>76,04 | H: | 7,45<br>7,52 | N: | 2,96<br>3,00 | O: | 13,51<br>13,62 |

## EXEMPLE XXXII

Préparation du N-p-hydroxyphényl [(tétraméthyl-5,5,8,8 tétrahydro-5,6,7,8 naphtyl-2) carbonyl]-6 naphtalène carboxamide-2.

Composé de formule III avec $R_1 = R_2 = R_3 = R_4 = -CH_3$ , R' et R'' = oxo et R = -CONH $C_6H_4$ p OH.

A une suspension de 0,9g (2,33 mmoles) d'acide [(tétraméthyl-5,5,8,8 tétrahydro-5,6,7,8 naphtyl-2) carbonyl]-6 naphtalène carboxylique-2 obtenu selon l'exemple VII dans 9cm$^3$ de dichlorométhane anhydre, on ajoute 490mg (3,03 mmoles) de N,N'-carbonyldiimidazole. Après 1h d'agitation à température ambiante, on ajoute 1cm$^3$ de N,N-diméthylformamide pur 0,31g (2,8 mmoles) de p-aminophénol à la solution obtenue. Le milieu réactionnel est agité une nuit à température ambiante. L'acide de départ n'ayant pas totalement réagi, on ajoute 0,1g de N,N'-carbonyldiimidazole puis 0,1g de p-aminophénol et agite 2h à température ambiante. Le milieu réactionnel est alors dilué par 50cm$^3$ de dichlorométhane lavé à l'acide chlorhydrique dilué puis à l'eau jusqu'à neutralité. La phase dichlorométhane est séchée sur sulfate de sodium puis concentrée sous pression réduite. L'amide brut est purifié par chromatographie sur gel de silice 60 dans un mélange toluène/dichlorométhane/acétate d'éthyle 5/3/2. Par recristallisation dans un mélange éther isopropylique/acétone, on obtient 0,33g de cristaux blancs de N-p-hydroxyphényl [(tétraméthyl-5,5,8,8 tétrahydro-5,6,7,8 naphtyl-2) carbonyl]-6 naphtalène carboxamide-2 dont le point de fusion est de 268-9 °C.

Le spectre R.M.N. $^1$H 250MHz est conforme à la structure attendue.

| Analyse élémentaire: $C_{32}H_{31}NO_3$ | | | | | | |
|---|---|---|---|---|---|---|
| Calculé:<br>Trouvé: | C: | 80,47<br>80,55 | H: | 6,54<br>6,27 | N: | 2,93<br>2,89 |

Au cours de la chromatographie sur silice, on isole également un deuxième produit qui par recristallisation dans un mélange acétone/méthanol fournit 0,3g de cristaux blancs de N,O-bis [(tétraméthyl-5,5,8,8 tétrahydro-5,6,7,8 naphtyl)-2 dicarbonyl-2,6 naphtyl] amino-4 phénol dont le point de fusion est de 239-240 °C.

Le spectre R.M.N. $^1$H 250MHz est conforme à la structure décrite.

| Analyse élémentaire: $C_{58}H_{55}NO_5$ | | | | | | | | |
|---|---|---|---|---|---|---|---|---|
| Calculé:<br>Trouvé: | C: | 82,33<br>82,13 | H: | 6,55<br>6,73 | N: | 1,66<br>1,65 | O: | 9,45<br>9,25 |

Ce produit décondensé est saponifié par de la potasse 6N en milieu hydroalcoolique pendant 5h à 50 °C. Après dilution à l'eau, évaporation de l'alcool sous pression réduite, et acidification par de l'acide chlorhydrique concentré, on obtient un mélange équimoléculaire de N-p-hydroxyphényl [(tétraméthyl-5,5,8,8 tétrahydro-5,6,7,8 naphtyl-2) carbonyl]-6 naphtalène carboxamide-2 et d'acide [(tétraméthyl-5,5,8,8 tétrahydro-5,6,7,8 naphtyl-2) carbonyl]-6 naphtalène carboxylique-2. Par chromatographie sur silice 60 dans un mélange toluène/dichlorométhane/acétate d'éthyle 5/3/2, on isole ainsi une deuxième fraction de 0,11g de N-p-hydroxyphényl [(tétraméthyl-5,5,8,8 tétrahydro-5,6,7,8 naphtyl-2) carbonyl]-6 naphtalène carboxamide-2 dont le point de fusion est identique à la précédente.

## EXEMPLE XXXIII

Préparation du N-(éthoxycarbonyl-1 méthylthio-3 propyl) [(tétraméthyl-5,5,8,8 tétrahydro-5,6,7,8 naphtyl-2) carbonyl]-6 naphtalène carboxamide-2.

Composé de formule III avec $R_1 = R_2 = R_3 = R_4 = -CH_3$ , R' et R'' = oxo et

$$R{=}{-}CONH{-}\underset{\displaystyle CO_2C_2H_5}{\underset{|}{CH}}{-}(CH_2)_2{-}S{-}CH_3.$$

On agite 1h30 à 40-50 °C, une solution de 1,2g (3,1 mmoles) d'acide [(tétraméthyl-5,5,8,8 tétrahydro-5,6,7,8 naphtyl-2) carbonyl]-6 naphtalène carboxylique-2 obtenu à l'exemple VII et de 605mg (3,72 mmoles) de N,N'-carbonyldiimidazole dans 15cm$^3$ de N,N-diméthyl formamide anhydre. On refroidit à 20 °C et ajoute 730mg (3,41 mmoles) de chlorhydrate de l'ester éthylique de la L-méthionine puis 0,48cm$^3$ (3,41 mmoles) de triéthylamine. Après 1h d'agitation à 20 °C puis 2h à 40-50 °C, le milieu réactionnel est versé sur

100cm$^3$ d'eau acidulée. On agite 15mn puis filtre le précipité qu'on lave alors abondamment à l'acide chlorhydrique dilué puis à l'eau. Après séchage sous vide à 70 °C, on purifie par chromatographie sur silice 60 dans un mélange toluène/dichlorométhane/acétate d'éthyle 5/3/2. Après évaporation, on obtient 0,8g de N-(carbéthoxy-1 méthylthio-3 propyl) [(tétraméthyl-5,5,8,8 tétrahydro-5,6,7,8 naphtyl-2) carbonyl]-6 naphtalène carboxamide-2 sous la forme d'une huile épaisse qui se transforme en produit vitreux après séchage prolongé sous vide à 70-80 °C.

Le spectre R.M.N. $^1$H 60MHz correspond à la structure attendue.

## EXEMPLE XXXIV

Préparation du N-(carboxy-1 méthylthio-3 propyl) [(tétraméthyl-5,5,8,8 tétrahydro-5,6,7,8 naphtyl-2) carbonyl]-6 naphtalène carboxamide-2.

Composé de formule III avec $R_1 = R_2 = R_3 = R_4 = -CH_3$ , R' et R'' = oxo et

$$R = -CONH-CH-(CH_2)_2-S-CH_3.$$
$$\qquad\qquad |$$
$$CO_2H$$

Une suspension de 0,58g (1 mmoles) de N-(éthoxycarbonyl-1 méthylthio-3 propyl) [(tétraméthyl-5,5,8,8 tétrahydro-5,6,7,8 naphtyl-2) carbonyl]-6 naphtalène carboxamide-2 obtenu à l'exemple XXXIII est agitée 30mn dans un mélange de 5cm$^3$ d'alcool et de 5cm$^3$ de potasse aqueuse 6N chauffé à 50 °C. Après addition de 50cm$^3$d'eau, l'alcool est éliminé par évaporation sous pression réduite à 40-45 °C. La phase aqueuse est alors acidifiée par 3cm$^3$ d'acide chlorhydrique 12N. Le précipité obtenu est essoré, lavé abondamment à l'eau et séché sur potasse à 70 °C puis repris à l'éther isopropylique tiède. Après refroidissement, filtration et séchage sous vide à 70 °C, on obtient 0,4g de cristaux blancs de N-(carboxy-1 méthylthio-3 propyl) [(tétraméthyl-5,5,8,8 tétrahydro-5,6,7,8 naphtyl-2) carbonyl-6 naphtalène carboxamide-2 dont le point de fusion est de 171-2 °C.

Le spectre R.M.N. $^1$H 250MHz est conforme à la structure attendue.

| Analyse élémentaire: $C_{31}H_{35}NO_4S$ 0,5 $H_2O$ | | | | | | | | | | |
|---|---|---|---|---|---|---|---|---|---|---|
| Calculé:<br>Trouvé: | C: | 70,69<br>70,95 | H: | 6,89<br>6,87 | N: | 2,66<br>2,59 | O: | 13,67<br>13,55 | S: | 6,08<br>5,98 |

## EXEMPLES DE COMPOSITION

### A. VOIE ORALE

Exemple 1 - Comprimé de 0,2g

- Acide (tétraméthyl-1,1,3,3 indanyl-5) carbonyl -6 naphtalène carboxylique-2....... 0,005g
- Amidon........................................ 0,110g
- Phosphate bicalcique......................... 0,020g
- Silice........................................ 0,020g
- Lactose....................................... 0,030g
- Talc.......................................... 0,010g
- Stéarate de magnésium........................ 0,005g

Dans cet exemple le composé actif peut être remplacé par la même quantité d'acide [(pentaméthyl-1,1,2,3,3 indanyl-5) carbonyl]-6 naphtalène carboxylique-2 ou d'acide [(méthano-5,8 tétrahydro-5,6,7,8 naphtyl-2) carbonyl]-6 naphtalène carboxylique-2 ou encore par 0,001g d'acide [(tétraméthyl-5,5,8,8 tétrahydro-5,6,7,8 naphtyl-2) carbonyl]-6 naphtalène carboxylique-2.

Exemple 2 - Suspension buvable en ampoules 5ml

| | |
|---|---|
| - Acide [(pentaméthyl-1,1,2,3,3 indanyl-5) hydroxyméthyl] -6 naphtalène carboxylique-2.. | 0,005g |
| - Glycérine.................................... | 0,500g |
| - Sorbitol à 70%............................... | 0,500g |
| - Saccharinate de sodium....................... | 0,010g |
| - Parahydroxybenzoate de méthyle............... | 0,040g |
| - Arôme........................................ | qs |
| - Eau purifiée qsp............................. | 5,000ml |

Dans cet exemple le composé actif peut être remplacé par la même quantité d'acide [(tétraméthyl-5,5,8,8 tétrahydro-5,6,7,8 naphtyl-2) hydroxyméthyl]-6 naphtalène carboxylique-2 ou par 0,001gde N-éthyl [-(tétraméthyl-5,5,8,8 tétrahydro-5,6,7,8 naphtyl-2) carbonyl]-6 naphtalène carboxamide-2.

B. VOIE TOPIQUE

Exemple 3 - Onguent

| | |
|---|---|
| - N-éthyl [(tétraméthyl-5,5,8,8 tétrahydro-5,6,7,8 naphtyl-2) hydroxyméthyl]-6 naphtalène carboxamide-2................................ | 0,100g |
| - Myristate d'isopropyle...................... | 81,620g |
| - Huile de vaseline fluide.................... | 9,100g |
| - Silice vendue par la Société DEGUSSA sous la dénomination de "Aérosil 200"................ | 9,180g |

Dans cet exemple le composé actif peut être remplacé par 0,005g d'acide [(tétraméthyl-1,1,3,3 indanyl-5) carbonyl]-6 naphtalène carboxylique-2 ou encore par 0,1g d'acide [(tétraméthyl-5,5,8,8 tétrahydro-5,6,7,8 naphtyl-2) hydroxyméthyl]-6 naphtalène carboxylique-2 ou de N-éthyl [(tétraméthyl-5,5,8,8 tétrahydro-5,6,7,8 naphtyl-2) carbonyl]-6 naphtalène carboxamide-2.

Exemple 4 - Crème anti-séborrhéique

- Stéarate de polyoxyéthylène (40 moles d'oxyde d'éthylène) vendu sous la dénomination de "Myrj 52"par la Société ATLAS................ 4,00g
- Mélange d'esters laurique de sorbitol et de sorbitan, polyoxyéthyléné à 20 moles d'oxyde d'éthylène vendu sous la dénomination de "Tween 20" par la Société ATLAS.............. 1,8g
- Mélange de mono et distéarate de glycérol vendu sous la dénomination de "GELEOL" par la Société GATTEFOSSE........................ 4,2g
- Propylèneglycol.............................. 10,0g
- Butylhydroxyanisole.......................... 0,01g
- Butylhydroxytoluène.......................... 0,02g
- Alcool céto-stéarylique...................... 6,2g
- Conservateurs................................ qs
- Perhydrosqualène............................. 18g
- Mélange de triglycérides caprylique-caprique vendu sous la dénomination de "Miglyol 812" par la Société DYNAMIT NOBEL................ 4,0g
- S-carboxyméthyl cystéine..................... 3,0g
- Triéthanolamine 99%.......................... 2,5g
- N-éthyl [(tétraméthyl-5,5,8,8 tétrahydro-5,6,7,8 naphtyl-2) hydroxyméthyl] -6 naphtalène carboxamide-2................................ 0,10g
- Eau qsp...................................... 100g

Exemple 5 - Crème anti-séborrhéique

- Stéarate de polyoxyéthylène (40 moles d'oxyde d'éthylène) vendu sous la dénomination de "Myrj 52" par la Société ATLAS............... 4,0g
- Mélange d'esters laurique de sorbitol et de sorbitan, polyoxyéthyléné à 20 moles d'oxyde d'éthylène, vendu sous la dénomination de "Tween 20" par la Société ATLAS.............. 1,8g

- Mélange de mono et distéarate de glycérol vendu sous la dénomination de "GELEOL" par la Société GATTEFOSSE.......................... 4,2g
- Propylèneglycol.............................. 10,0g
- Butylhydroxyanisole.......................... 0,01g
- Butylhydroxytoluène.......................... 0,02g
- Alcool céto-stéarylique...................... 6,2g
- Conservateurs................................ qsp
- Perhydrosqualène............................. 18,0g
- Mélange de triglycérides caprylique-caprique vendu sous la dénomination de "Miglyol 812" par la Société DYNAMIT NOBEL................. 4,0g
- Amino-5 carboxy-5 thia-3 pentanoate de benzylthio-2 éthylammonium.................. 3,0g
- Acide [(tétraméthyl-5,5,8,8 tétrahydro-5,6,7,8 naphtyl-2) hydroxyméthyl] -6 naphtalène carboxylique-2.................................. 0,1g
- Eau qsp...................................... 100g

Exemple 6 - Lotion pour les cheveux

- Propylèneglycol.............................. 20,0g
- Ethanol...................................... 34,87g
- Polyéthylèneglycol de masse moléculaire 400.. 40,0g
- Eau.......................................... 4,0g
- Butylhydroxyanisole.......................... 0,01g
- Butylhydroxytoluène.......................... 0,02g
- N-éthyl [(pentaméthyl-1,1,2,3,3 indanyl-2) carbonyl] -6 naphtalène carboxamide-2........ 0,10g
- Minoxidil.................................... 1,0g

Exemple 7 - Gel anti-acné

- N-éthyl [(tétraméthyl-1,1,3,3 indanyl-5) carbonyl]-6 naphtalène carboxamide-2........ 0,05g
- Alcool isopropylique........................ 40,0g
- Polymère de l'acide acrylique vendu sous la dénomination "CARBOPOL 940" par la Société GOODRICH CHEMICAL CO................. 1,0g
- Triéthanolamine 99%.......................... 0,6g
- Butylhydroxyanisole.......................... 0,01g
- Butylhydroxytoluène.......................... 0,02g
- Tioxolone.................................... 0,5g
- Propylèneglycol.............................. 8,0g
- Eau purifiée qsp............................. 100g

Dans cet exemple, le composé actif peut être remplacé par la même quantité de l'acide [(tétraméthyl-5,5,8,8 tétrahydro-5,6,7,8 naphtyl-2) hydroxyméthyl]-6 naphtalène carboxylique-2 ou par 0,1g de N-éthyl [-(tétraméthyl-5,5,8,8 tétrahydro-5,6,7,8 naphtyl-2) hydroxyméthyl]-6 naphtalène carboxamide-2 ou encore par 0,1g d'acide (tétraméthyl-5,5,8,8 tétrahydro-5,6,7,8 naphtyl-2)-1 (carboxy-6 naphtyl-2)-1 méthane.

Exemple 8 - Crème huile-dans-l'eau anionique

- Acide [(tétraméthyl-5,5,8,8 tétrahydro-5,6,7,8 naphtyl-2) carbonyl]-6 naphtalène carboxylique-2............................. 0,010g
- Dodécyl sulfate de sodium.................. 0,800g
- Glycérol................................... 2,000g
- Alcool stéarylique......................... 20,000g
- Triglycérides d'acides caprique/caprylique vendus par la Société Dynamit Nobel sous le nom de "Miglyol 812"........................ 20,000g
- Conservateurs.............................. qs
- Eau déminéralisée.......................... 100,000g

Exemple 9 - Gel

- Acide [(méthano-5,8 tétrahydro-5,6,7,8 naphtyl-2) carbonyl]-6 naphtalène carboxylique-2............................ 0,050g
- Hydroxypropyl cellulose vendue par la Société Hercules sous le nom de "Klucel HF". 2,000g
- Eau/éthanol (50:50) qsp.................... 100,000g

## Revendications

### Revendications pour les Etats contractants suivants: BE, CH, DE, FR, GB, IT, LI, NL, SE

**1.** Composés bicycliques naphtaléniques, caractérisés par le fait qu'ils répondent à la formule suivante:

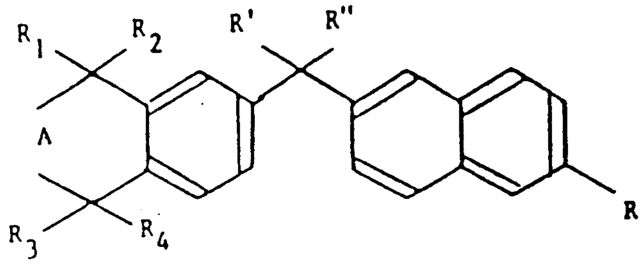

(I)

dans laquelle:

A représente un radical méthylène ou diméthylène, substitués ou non par un radical alkyle inférieur,

$R_1$ , $R_2$ , $R_3$ et $R_4$ , identiques ou différents, représentent un atome d'hydrogène ou un radical alkyle inférieur ayant de 1 à 6 atomes de carbone,

ou $R_1$ et $R_3$ ,pris ensemble, forment un pont méthylène ou diméthylène, quand A représente un radical diméthylène,

R' représente un atome d'hydrogène, un radical OH, un radical alkoxy ayant de 1 à 4 atomes de carbone, un radical acyloxy ayant de 1 à 4 atomes de carbone ou un radical amino,

R'' représente un atome d'hydrogène ou un radical alkoxy ayant de 1 à 4 atomes de carbone,

ou R' et R'' , pris ensemble, forment un radical oxo (=O), méthano (=$CH_2$) ou hydroxyimino (=N-OH),

R représente le radical $-CH_2OH$ ou le radical $-COR_5$,

$R_5$ représentant un atome d'hydrogène, le radical $OR_6$ ou le radical

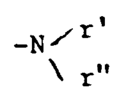

$R_6$ représentant un atome d'hydrogène, un radical alkyle ayant de 1 à 20 atomes de carbone, monohydroxyalkyle, polyhydroxyalkyle, aryle ou aralkyle éventuellement substitué(s) ou un reste d'un sucre ou encore le radical

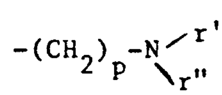

p étant 1, 2 ou 3 et r' et r'', identiques ou différents, représentant un atome d'hydrogène, un radical

alkyle inférieur, un radical monohydroxyalkyle éventuellement interrompu par un hétéroatome, un radical polyhydroxyalkyle, un radical aryle ou benzyle éventuellement substitué(s), un reste d'aminoacide, d'aminoester ou de sucre aminé ou pris ensemble forment, avec l'atome d'azote auquel ils sont rattachés, un hétérocycle substitué ou non,
et les sels desdits composés de formule (I) ainsi que leurs isomères optiques.

2. Composés selon la revendication 1, caractérisés par le fait que les radicaux alkyles inférieurs et ceux ayant jusqu'à 20 atomes de carbone sont pris dans le groupe constitué par les radicaux méthyle, éthyle, propyle, isopropyle, butyle, tertiobutyle, éthyl-2 hexyle, isooctyle, octyle, dodécyle, hexadécyle et octadécyle.

3. Composés selon la revendication 1, caractérisés par le fait que le radical monohydroxyalkyle est le radical hydroxy-2 éthyle, hydroxy-2 propyle ou hydroxy-2' éthoxy-2 éthyle.

4. Composés selon la revendication 1, caractérisés par le fait que le radical polyhydroxyalkyle est le radical dihydroxy-2,3 propyle, dihydroxy-1,3 propyl-2 ou le reste du pentaérythritol.

5. Composés selon la revendication 1, caractérisés par le fait que le radical alkoxy est le radical méthoxy, isopropoxy, butoxy ou tert-butoxy.

6. Composés selon la revendication 1, caractérisés par le fait que le radical aryle est un radical phényle éventuellement substitué par un atome d'halogène, -OH, $-NO_2$ , un radical alkyle inférieur, un radical trifluorométhyle ou une fonction acide carboxylique.

7. Composés selon la revendication 1, caractérisés par le fait que le radical aralkyle est le radical benzyle ou phénéthyle.

8. Composés selon la revendication 1, caractérisés par le fait que le reste d'un sucre dérive du glucose, du mannose, de l'érythrose ou du galactose.

9. Composés selon la revendication 1, caractérisés par le fait que le reste de sucres aminés dérive de glucosamine, de galactosamine, de mannosamine ou de méglumine.

10. Composés selon la revendication 1, caractérisés par le fait que les radicaux r' et r'' pris ensemble forment, avec un atome d'azote auquel ils sont rattachés, un radical pipéridino, pipérazino, morpholino, pyrrolidino ou (hydroxy-2'éthyl)-4 pipérazino.

11. Composés selon l'une quelconque des revendications 1 à 10, caractérisés par le fait qu'ils répondent à la formule suivante:

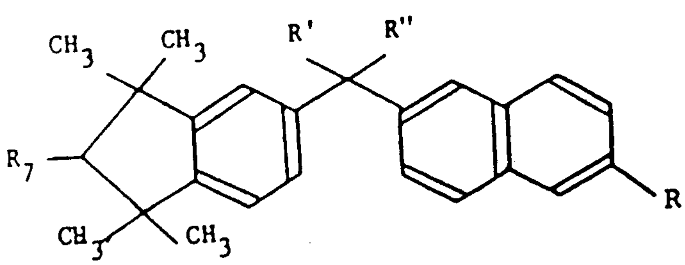

(II)

dans laquelle:

R' représente un atome d'hydrogène ou un radical OH,

R'' représente un atome d'hydrogène,

ou R' et R'' pris ensemble forment un radical oxo (=O),

R représente le radical $-CH_2OH$ ou le radical $-COOR_6$, $R_6$ représentant un atome d'hydrogène ou un radical alkyle ayant de 1 à 6 atomes de carbone,

et $R_7$ représente un atome d'hydrogène ou un radical méthyle.

**12.** Composés selon l'une quelconque des revendications 1 à 10, caractérisés par le fait qu'ils répondent à la formule suivante:

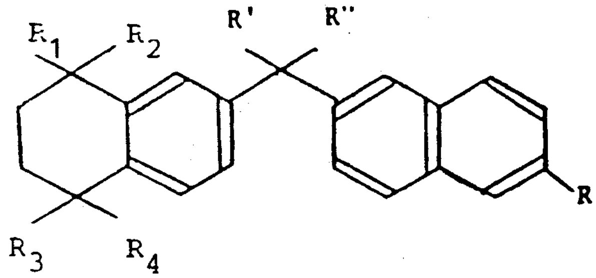

(III)

dans laquelle:

$R_1$, $R_2$, $R_3$ et $R_4$ représentent le radical $-CH_3$,

R' représente un atome d'hydrogène, un radical OH, un radical alkoxy ayant de 1 à 4 atomes de carbone, un radical acyloxy ayant de 1 à 4 atomes de carbone,

R'' représente un atome d'hydrogène,

ou R' et R'' pris ensemble forment un radical oxo ( = O) ou un radical méthano ( $= CH_2$),

et R représente le radical $-CH_2OH$ ou le radical $-COR_5$,

$R_5$ représentant un atome d'hydrogène, le radical $-OR_6$ ou le radical

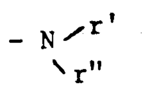 ,

$R_6$ représentant un atome d'hydrogène ou un radical alkyle ayant de 1 à 6 atomes de carbone,

et r' ou r'' représentant un atome d'hydrogène, un radical alkyle ayant de 1 à 6 atomes de carbone, hydroxy-4 phényle, hydroxy-2'-éthoxy-2 éthyle ou carboxy-1 méthylthio-3 propyle.

**13.** Composés selon l'une quelconque des revendications 1 à 10, caractérisés par le fait qu'ils répondent à la formule suivante:

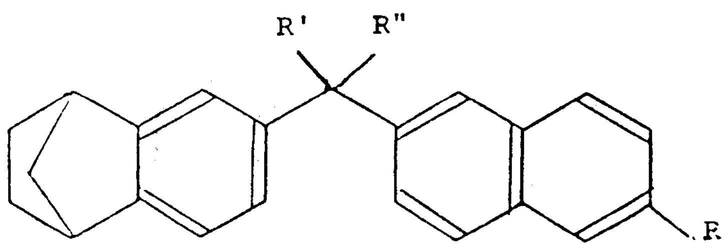

(IV)

dans laquelle:

R' et R'' pris ensemble forment un radical oxo ( = O)

R représente-$COR_5$,

$R_5$ représentant le radical $-OR_6$ ou le radical

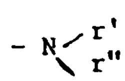

$R_6$ représentant un atome d'hydrogène ou un radical alkyle ayant de 1 à 6 atomes de carbone,

et r' et r'' représentant un atome d'hydrogène ou un radical alkyle ayant de 1 à 6 atomes de carbone.

**14.** Composés selon l'une quelconque des revendications 1 à 13, caractérisés par le fait qu'ils sont pris

dans le groupe constitué par:
[(tétraméthyl-1,1,3,3 indanyl-5) carbonyl]-6 naphtalène carboxylate de méthyle-2,
Acide [(tétraméthyl-1,1,3,3 indanyl-5) carbonyl]-6 naphtalène carboxylique-2,
[(pentaméthyl-1,1,2,3,3 indanyl-5) carbonyl]-6 naphtalène carboxylate de méthyle-2,
Acide [(pentaméthyl-1,1,2,3,3 indanyl-5) carbonyl]-6 naphtalène carboxylique-2,
Acide [(pentaméthyl-1,1,2,3,3 indanyl-5) hydroxyméthyl]-6 naphtalène carboxylique-2,
N-éthyl [(tétraméthyl-1,1,3,3 indanyl-5) carbonyl]-6 naphtalène carboxamide-2,
N-éthyl [(pentaméthyl-1,1,2,3,3 indanyl-5) carbonyl]-6 naphtalène carboxamide-2, et
N-éthyl [(pentaméthyl-1,1,2,3,3 indanyl-5) hydroxyméthyl]-6 naphtalène carboxamide-2.

**15.** Composés selon l'une quelconque des revendications 1 à 13, caractérisés par le fait qu'ils sont pris dans le groupe constitué par:
Acide [(tétraméthyl-5,5,8,8 tétrahydro-5,6,7,8 naphtyl-2) hydroxyméthyl]-6 naphtalène carboxylique-2,
N-éthyl [(tétraméthyl-5,5,8,8 tétrahydro-5,6,7,8 naphtyl-2) hydroxyméthyl]-6 naphtalène carboxamide-2,
(tétraméthyl-5,5,8,8 tétrahydro-5,6,7,8 naphtyl-2)-1 (carboxy-6 naphtyl -2)-1 méthane,
Acide [(tétraméthyl-5,5,8,8 tétrahydro-5,6,7,8 naphtyl-2) acétoxyméthyl]-6 naphtalène carboxylique-2,
[(tétraméthyl-5,5,8,8 tétrahydro-5,6,7,8 naphtyl-2)-2 éthényl-2]-6 naphtalène carboxylate de méthyle-2,
Acide [(tétraméthyl-5,5,8,8 tétrahydro-5,6,7,8 naphtyl-2)-2 éthényl-2]-6 naphtalène carboxylique-2,
[(tetraméthyl-5,5,8,8 tétrahydro-5,6,7,8 naphtyl-2) hydroxyméthyl]-6 naphtalène carbinol-2,
[(tétraméthyl-5,5,8,8 tétrahydro-5,6,7,8 naphtyl-2) butoxyméthyl]-6 naphtalène carbinol-2,
Acide [(tétraméthyl-5,5,8,8 tétrahydro-5,6,7,8 naphtyl-2) butoxyméthyl]-6 naphtalène carboxylique-2,
[(tétraméthyl-5,5,8,8 tétrahydro-5,6,7,8 naphtyl-2) hydroxyméthyl]-6 naphtaldéhyde-2,
[(tétraméthyl-5,5,8,8 tétrahydro-5,6,7,8 naphtyl-2) hydroxyiminométhyl]-6 naphtalène carboxylate d'éthyle-2,
Acide [(tétraméthyl-5,5,8,8 tétrahydro-5,6,7,8 naphtyl-2) hydroxyiminométhyl]-6 naphtalène carboxylique-2,
N-éthyl [(tétraméthyl-5,5,8,8 tétrahydro-5,6,7,8 naphtyl-2) hydroxyiminométhyl]-6 naphtalène carboxamide-2,
[(tétraméthyl-5,5,8,8 tétrahydro-5,6,7,8 naphtyl-2) aminométhyl]-6 naphtalène carboxylate d'éthyle-2,
Acide [(tétraméthyl-5,5,8,8 tétrahydro-5,6,7,8 naphtyl-2) aminométhyl]-6 naphtalène carboxylique-2,
Acide [(tétraméthyl-5,5,8,8 tétrahydro-5,6,7,8 naphtyl-2) carbonyl]-6 naphtalène carboxylique-2 et son ester méthylique
N-éthyl [(tétraméthyl-5,5,8,8 tétrahydro-5,6,7,8 naphtyl-2) carbonyl]-6 naphtalène carboxamide-2,
[(tétraméthyl-5,5,8,8 tétrahydro-5,6,7,8 naphtyl-2) carbonyl]-6 naphtalène carboxaldéhyde-2,
[(tétraméthyl-5,5,8,8 tétrahydro-5,6,7,8 naphtyl-2) carbonyl]-6 naphtalène carbinol-2,
N(-hydroxy-2' éthoxy-2 éthyl) [(tétraméthyl-5,5,8,8 tétrahydro-5,6,7,8 naphtyl-2) carbonyl]-6 naphtalène carboxamide-2,
N-p-hydroxyphényl [(tétraméthyl-5,5,8,8 tétrahydro-5,6,7,8 naphtyl-2) carbonyl]-6 naphtalène carboxamide-2,
N-(éthoxycarbonyl-1 méthylthio-3 propyl) [(tétraméthyl-5,5,8,8 tétrahydro-5,6,7,8 naphtyl-2) carbonyl]-6 naphtalène carboxamide-2,
N-(carboxy-1 méthylthio-3 propyl) [(tétraméthyl-5,5,8,8 tétrahydro-5,6,7,8 naphtyl-2) carbonyl]-6 naphtalène carboxamide-2,
Acide [(tétrahydro-5,6,7,8 naphtyl-2) carbonyl]-6 naphtalène carboxylique-2 et son ester méthylique,
N-éthyl [(tétrahydro-5,6,7,8 naphtyl-2) carbonyl]-6 naphtalène carboxamide-2.

**16.** **Composé** selon l'une quelconque des revendications 1 à 13, caractérisé par le fait qu'il est l'acide [-(méthano-5,8 tétrahydro-5,6,7,8 naphtyl-2) carbonyl]-6 naphtalène carboxylique-2 et son ester méthylique.

**17.** Procédé de préparation des composés selon l'une quelconque des revendications 1 à 16, caractérisé par le fait qu'il consiste à faire réagir en milieu solvant organique dans les conditions de la réaction de Friedel Crafts un halogénure tel qu'un chlorure d'acide correspondant à la formule suivante:

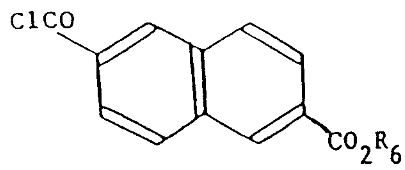

sur un composé naphtalénique de formule:

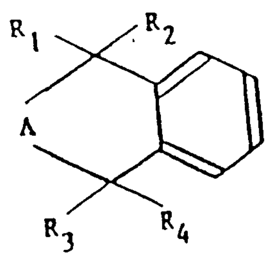

dans lesquels:

A, $R_1$ à $R_4$ ont les mêmes significations que celles données à la revendication 1, $R_6$ étant un radical alkyle ayant de 1 à 20 atomes de carbone, que l'on procède, si nécessaire à la saponification du céto-ester obtenu en céto-acide correspondant et à la transformation subséquente dudit céto-acide en amide correspondant par action d'une amine de formule:

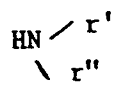

dans laquelle:

r' et r'' ont les mêmes significations que celles données à la revendication 1, ou à la transformation subséquente dudit céto-acide en hydroxyacide, en méthanoacide, en hydroxyiminoacide, en méthylène acide ou en diol et à l'oxydation éventuelle du diol en alcool aldéhyde ou en céto-aldéhyde correspondants ou à sa transformation éventuelle en dérivés acyloxy ou alkoxy.

**18.** Procédé selon la revendication 17, caractérisé par le fait que la réaction de condensation est effectuée en présence de chlorure d'aluminium anhydre dans du dichloro-1,2 éthane à une température comprise entre 0 et 25° C sous agitation.

**19.** Procédé selon la revendication 17, caractérisé par le fait que la préparation de l'amide est effectuée en présence de N,N'-carbonyl diimidazole.

**20.** Procédé selon la revendication 17, caractérisé par le fait que la réduction du céto-acide en hydroxy-acide correspondant est effectuée en présence du borohydrure de sodium dans le T.H.F.

**21.** Procédé selon la revendication 17 , caractérisé par le fait que l'alcool aldéhyde et le céto-aldéhyde sont obtenus par oxydation du diol à l'aide de chlorochromate de pyridinium, ledit diol correspondant résultant d'une réaction de réduction du céto-acide en présence d'hydrure de lithium aluminium.

**22.** Procédé selon la revendication 17 , caractérisé par le fait que les composés de formule I dans laquelle R' = R'' = H sont obtenus par réduction au zinc des dérivés cétoniques correspondants dans l'acide acétique en présence d'acide Chlorhydrique.

**23.** Procédé selon la revendication 17, caractérisé par le fait que les composés de formule I dans laquelle R' et R'' = méthano ( = $CH_2$) sont obtenus par réaction des composés cétoniques correspondants avec un mélange de bromure de triphényl méthyl phosphonium et d'amidure de sodium.

**24.** Procédé selon la revendication 17, caractérisé par le fait que les composés de formule I dans laquelle R' et R" = hydroxyimino ( = N-OH) sont obtenus par réaction des composés cétoniques avec du chlorhydrate d'hydroxylamine dans un solvant organique et en présence d'une base.

**25.** Médicament, caractérise par le fait qu'il est un composé de formule (I), selon l'une quelconque des revendications 1 à 16 ou obtenu selon l'une quelconque des revendications 17 à 24.

**26.** Médicament selon la revendication 25 , caractérisé par le fait qu'il est administré à une dose journalière d'environ 2µg/kg à 2mg/kg de poids corporel.

**27.** Composition pharmaceutique, caractérisée par le fait qu'elle contient dans un véhicule approprié, pour une administration par voie entérale, parentérale, topique ou oculaire, au moins un composé de formule (I) selon l'une quelconque des revendications 1 à 16, ou obtenu selon l'une quelconque des revendications 17 à 24.

**28.** Composition selon la revendication 27, caractérisée par le fait qu'elle se présente sous une forme appropriée pour une application topique ou oculaire et contient de 0,0005 à environ 5% en poids d'un composé de formule (I).

**29.** Utilisation d'un composé, selon l'une quelconque des revendications 1 à 16 ou obtenu selon l'une quelconque des revendications 17 à 24, pour la préparation d'une composition pharmaceutique destinée au traitement des affections dermatologiques, respiratoires ainsi qu'ophtalmologiques.

**30.** Composition cosmétique pour l'hygiène corporelle et capillaire, caractérisée par le fait qu'elle contient dans un véhicule cosmétique approprié, au moins un composé de formule (I) selon l'une quelconque des revendications 1 à 16 ou obtenu selon l'une quelconque des revendications 17 à 24.

**31.** Composition cosmétique selon la revendication 30, caractérisée par le fait qu'elle contient le composé de formule (I) à une concentration comprise entre 0,0005 et 2% et de préférence entre 0,01 et 1% en poids.

**Revendications pour les Etats contractants suivants: AT, ES, GR**

**1.** Procédé de préparation d'un composé bicyclique naphtalénique répondant à la formule générale suivante :

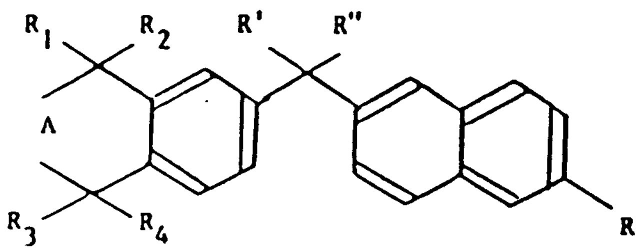

(I)

dans laquelle :

A représente un radical méthylène ou diméthylène, susbtitués ou non par un radical alkyle inférieur,

$R_1$, $R_2$, $R_3$ et $R_4$, identiques ou différents, représentent un atome d'hydrogène ou un radical alkyle inférieur ayant de 1 à 6 atomes de carbone,

ou $R_1$ et $R_3$, pris ensemble, forment un pont méthylène ou diméthylène, quand A représente un radical diméthylène,

R' représente un atome d'hydrogène, un radical OH, un radical alkoxy ayant de 1 à 4 atomes de carbone, un radical acyloxy ayant de 1 à 4 atomes de carbone ou un radical amino,

R" représente un atome d'hydrogène ou un radical alkoxy ayant de 1 à 4 atomes de carbone,

ou R' et R", pris ensemble, forment un radical oxo ( = O), méthano ( = $CH_2$) ou hydroxyimino ( = N-OH ),

R représente le radical $-CH_2OH$ ou le radical $-COR_5$,
$R_5$ représentant un atome d'hydrogène, le radical $OR_6$ ou le radical

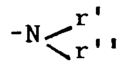

$R_6$ représentant un atome d'hydrogène, un radical alkyle ayant de 1 à 20 atomes de carbone, monohydroxyalkyle, polyhydroxyalkyle, aryle ou aralkyle éventuellement substitué(s) ou un reste d'un sucre ou encore le radical

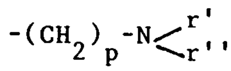

p étant 1, 2 ou 3 et r' et r'', identiques ou différents, représentant un atome d'hydrogène, un radical alkyle inférieur, un radical monohydroxyalkyle éventuellement interrompu par un hétéroatome, un radical polyhydroxyalkyle, un radical aryle ou benzyle éventuellement substitué(s), un reste d'aminoacide, d'aminoester ou de sucre aminé ou pris ensemble forment, avec l'atome d'azote auquel ils sont rattachés, un hétérocycle substitué ou non,
ou l'un de ses sels et isomères optiques, caractérisé par le fait qu'il consiste à faire réagir en milieu solvant organique dans les conditions de la réaction de Friedel Crafts un halogénure tel qu'un chlorure d'acide correspondant à la formule suivante :

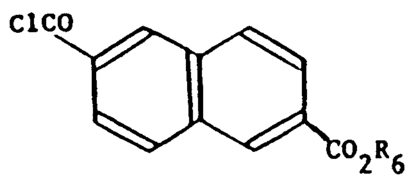

sur un composé naphtalénique de formule :

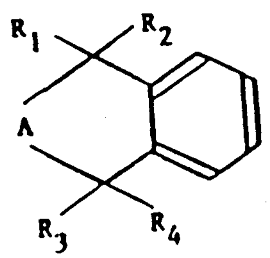

dans lesquels :
A, $R_1$ à $R_4$ ont les mêmes significations que celles données ci-dessus, $R_6$ étant un radical alkyle ayant de 1 à 20 atomes de carbone, que l'on procède, si nécessaire à la saponification du céto-ester obtenu en céto-acide correspondant et à la transformation subséquente dudit céto-acide en amide correspondant par action d'une amine de formule :

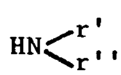

dans laquelle :
r' et r'' ont les mêmes significations que celles données ci-dessus, ou à la transformation subséquente dudit céto-acide en hydroxyacide, en méthanoacide, en hydroxyiminoacide, en méthylène acide ou en diol et à l'oxydation éventuelle du diol en alcool aldéhyde ou en céto-aldéhyde

correspondants ou à sa transformation éventuelle en dérivés acyloxy ou alkoxy.

2. Procédé selon la revendication 1, caractérisé par le fait que la réaction de condensation est effectuée en présence de chlorure d'aluminium anhydre dans du dichloro-1,2 éthane à une température comprise entre 0 et 25 °C sous agitation.

3. Procédé selon la revendication 1, caractérisé par le fait que la préparation de l'amide est effectuée en présence de N,N'-carbonyl diimidazole.

4. Procédé selon la revendication 1, caractérisé par le fait que la réduction du céto-acide en hydroxy-acide correspondant est effectuée en présence du borohydrure de sodium dans le T.H.F.

5. Procédé selon la revendication 1, caractérisé par le fait que l'alcool aldéhyde et le céto-aldéhyde sont obtenus par oxydation du diol à l'aide de chlorochromate de pyridinium, ledit diol correspondant résultant d'une réaction de réduction du céto-acide en présence d'hydrure de lithium aluminium.

6. Procédé selon la revendication 1, caractérisé par le fait que les composés de formule I dans laquelle R' = R'' = H sont obtenus par réduction au zinc des dérivés cétoniques correspondants dans l'acide acétique en présence d'acide sulfurique.

7. Procédé selon la revendication 1, caractérisé par le fait que les composés de formule I dans laquelle R' et R'' = méthano ( = $CH_2$) sont obtenus par réaction des composés cétoniques correspondants avec un mélange de bromure de triphényl méthyl phosphonium et d'amidure de sodium.

8. Procédé selon la revendication 1, caractérisé par le fait que les composés de formule I dans laquelle R' et R'' = hydroxyimino ( = N-OH) sont obtenus par réaction des composés cétoniques avec du chlorhydrate d'hydroxylamine dans un solvant organique et en présence d'une base.

9. Utilisation d'un composé obtenu selon l'une quelconque des revendications 1 à 8 pour la préparation d'une composition pharmaceutique destinée au traitement des affections dermatologiques, respiratoires ainsi qu'ophtalmologiques.

**Claims**

**Claims for the following Contracting States: BE, CH, DE, FR, GB, IT, LI, NL, SE**

1. Bicyclic naphthalene compounds, characterised in that they correspond to the following formula:

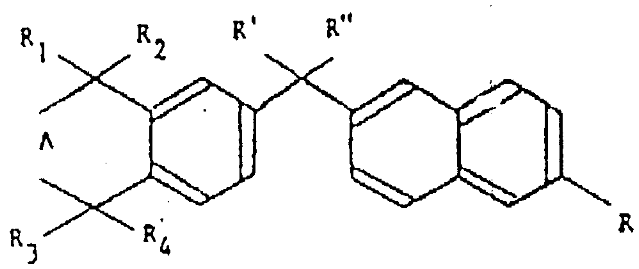

(I)

in which:

A denotes a methylene or dimethylene radical, substituted or unsubstituted with a lower alkyl radical,

$R_1$, $R_2$, $R_3$ and $R_4$, which may be identical or different, denote a hydrogen atom or a lower alkyl radical having from 1 to 6 carbon atoms,

or $R_1$ and $R_3$, taken together, form a methylene or dimethylene bridge, when A denotes a dimethylene radical,

R' denotes a hydrogen atom, an OH radical, an alkoxy radial having from 1 to 4 carbon atoms, an acyloxy radical having from 1 to 4 carbon atoms or an amino radial,

R'' denotes a hydrogen atom or an alkoxy radical having from 1 to 4 carbon atoms,

or R' and R'', taken together, form an oxo ( = O), methano ( = $CH_2$) or hydroxyimino ( = N-OH)

radical, and

R denotes a $-CH_2OH$ radical or a radical $-COR_5$,

$R_5$ denoting a hydrogen atom, a radical $OR_6$ or a radical

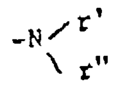

$R_6$ denoting a hydrogen atom, an alkyl radical having from 1 to 20 carbon atoms, a monohydroxyalkyl, polyhydroxyalky, aryl or aralkyl radical, these radicals optionally being substituted, or a residue of a sugar or alternatively a radical

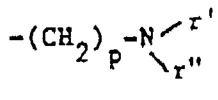

p being 1, 2 or 3 and r' and r'', which may be identical or different, denoting a hydrogen atom, a lower alkyl radical, a monohydroxyalkyl radical optionally interrupted by a hetero atom, a polyhydroxyalkyl radical, an optionally substituted aryl or benzyl radical, an amino acid residue, amino ester residue or amino sugar residue or, taken together, form, with the nitrogen atom to which they are attached, a substituted or unsubstituted heterocycle,

and the salts of the said compounds of the formula (I) as well as their optical isomers.

**2.** Compounds according to Claim 1, characterised in that the lower alkyl radicals and those having up to 20 carbon atoms are selected from the group consisting of methyl, ethyl, propyl, isopropyl, butyl, tert-butyl, 2-ethylhexyl, isooctyl, octyl, dodecyl, hexadecyl and octadecyl radicals.

**3.** Compounds according to Claim 1, characterised in that the monohydroxyalkyl radical is a 2-hydroxyethyl, 2-hydroxypropyl or 2'-hydroxy-2-ethoxyethyl radical.

**4.** Compounds according to Claim 1, characterised in that the polyhydroxyalkyl radical is a 2,3-dihydroxypropyl or 1,3-dihydroxy-2-propyl radical or a pentaerythritol residue.

**5.** Compounds according to Claim 1, characterised in that the alkoxy radical is a methoxy isopropoxy, butoxy or tert-butoxy radical.

**6.** Compounds according to Claim 1, characterised in that the aryl radical is a phenyl radical optionally substituted with a halogen atom, -OH, $-NO_2$, a lower alkyl radical, a trifluoromethyl radical or a carboxylic acid group.

**7.** Compounds according to Claim 1, characterised in that the aralkyl radical is a benzyl or phenethyl radical.

**8.** Compounds according to Claim 1, characterised in that the residue of a sugar is derived from glucose, from mannose, from erythrose or from galactose.

**9.** Compounds according to Claim 1, characterised in that the amino sugar residue is derived from glucosamine, from galactosamine, from mannosamine or from meglumine.

**10.** Compounds according to Claim 1, characterised in that the radicals r' and r'', taken together, form, with a nitrogen atom to which they are attached, a piperidino, piperazino, morpholino, pyrrolidino or 4-(2'-hydroxyethyl)piperazino radical.

**11.** Compounds according to any one of claims 1 to 10, characterised in that they correspond to the following formula:

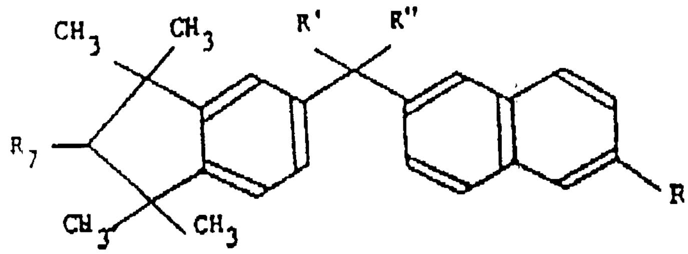

(II)

in which:

R' denotes a hydrogen atom or an OH radical,

R'' denotes a hydrogen atom,

or R' and R'', taken together, form an oxo (=O) radical,

R denotes a $-CH_2OH$ radical or a radical $-COOR_6$, $R_6$ denoting a hydrogen atom or an alkyl radical having from 1 to 6 carbon atoms,

and $R_7$ denotes a hydrogen atom or a methyl radical.

**12.** Compounds according to any one of Claims 1 to 10, characterised in that they correspond to the following formula:

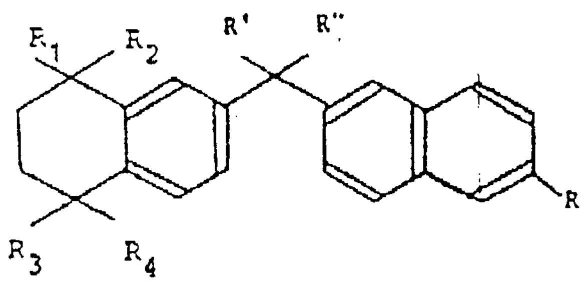

(III)

in which:

$R_1$, $R_2$, $R_3$ and $R_4$ denote a $-CH_3$ radical,

R' denotes a hydrogen atom, an OH radical, an alkoxy radical having from 1 to 4 carbon atoms or an acyloxy radical having from 1 to 4 carbon atoms,

R'' denotes a hydrogen atom,

or R' and R'', taken together form an oxo (=O) radical or a methano ($=CH_2$) radical,

and R denotes a $-CH_2OH$ radical or a radical $-COR_5$,

$R_5$ denoting a hydrogen atom, a radical $-OR_6$ or a radical

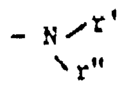

$R_6$ denoting a hydrogen atom or an alkyl radical having from 1 to 6 carbon atoms,

and r' or r'' denoting a hydrogen atom, an alkyl radical having from 1 to 6 carbon atoms or a 4-hydroxyphenyl, 2'-hydroxy-2-ethoxyethyl or 1-carboxy-3-(methylthio)propyl radical.

**13.** Compounds according to any one of Claims 1 to 10, characterised in that they correspond to the following formula:

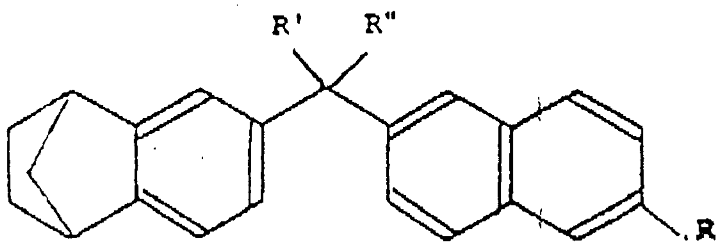

(IV)

in which:

R' and R'', taken together, form an oxo (=O) radical,

R denotes $-COR_5$,

$R_5$ denoting a radical $-OR_6$ or a radical

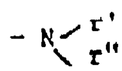

$R_6$ denoting a hydrogen atom or an alkyl radical having from 1 to 6 carbon atoms,

and r' and r'' denoting a hydrogen atom or an alkyl radical having from 1 to 6 carbon atoms.

**14.** Compounds according to any one of Claims 1 to 13, characterised in that they are selected from the group consisting of:

methyl 6-[(1,1,3,3-tetramethyl-5-indanyl)carbonyl]-2-naphthalenecarboxylate,
6-[(1,1,3,3-tetramethyl-5-indanyl)carbonyl]-2-naphthalenecarboxylic acid,
methyl 6-[(1,1,2,3,3-pentamethyl-5-indanyl)carbonyl]-2-naphthalenecarboxylate,
6-[(1,1,2,3,3-pentamethyl-5-indanyl)carbonyl]-2-naphthalenecarboxylic acid,
6-[(1,1,2,3,3-pentamethyl-5-indanyl)hydroxymethyl]- 2-naphthalenecarboxylic acid,
N-ethyl-6-[(1,1,3,3-tetramethyl-5-indanyl)carbonyl]-2-naphthalenecarboxamide,
N-ethyl-6-[(1,1,2,3,3-pentamethyl-5-indanyl)carbonyl]-2-naphthalenecarboxamide, and
N-ethyl-6-[(1,1,2,3,3-pentamethyl-5-indanyl)hydroxymethyl]-2-naphthalenecarboxamide.

**15.** Compounds according to any one of Claims 1 to 13, characterised in that they are selected from the group consisting of:

6-[(5,5,8,8-tetramethyl-5,6,7,8-tetrahydro-2-naphthyl)hydroxymethyl]-2-naphthalenecarboxylic acid,
N-ethyl-6-[(5,5,8,8-tetramethyl-5,6,7,8-tetrahydro-2-naphthyl)hydroxymethyl]-2-naphthalenecarboxamide,
1-(5,5,8,8-tetramethyl-5,6,7,8-tetrahydro-2-naphthyl)-1-(6-carboxy-2-naphthyl)methane,
6-[(5,5,8,8-tetramethyl-5,6,7,8-tetrahydro-2-naphthyl)acetoxymethyl]-2-naphthalenecarboxylic acid,
methyl 6-[2-(5,5,8,8-tetramethyl-5,6,7,8-tetrahydro-2-naphthyl)-2-ethenyl]-2-naphthalenecarboxylate,
6-[2-(5,5,8,8-tetramethyl-5,6,7,8-tetrahydro-2-naphthyl)-2-ethenyl]-2-naphthalenecarboxylic acid,
6-[(5,5,8,8-tetramethyl-5,6,7,8-tetrahydro-2-naphthyl)hydroxymethyl]-2-naphthalenecarbinol,
6-[(5,5,8,8-tetramethyl-5,6,7,8-tetrahydro-2-naphthyl)butoxymethyl]-2-naphthalenecarbinol,
6-[(5,5,8,8-tetramethyl-5,6,7,8-tetrahydro-2-naphthyl)butoxymethyl]-2-naphthalenecarboxylic acid,
6-[(5,5,8,8-tetramethyl-5,6,7,8-tetrahydro-2-naphthyl)hydroxymethyl]-2-naphthaldehyde,
ethyl 6-[(5,5,8,8-tetramethyl-5,6,7,8-tetrahydro-2-naphthyl)hydroxyiminomethyl]-2-naphthalenecarboxylate,
6-[(5,5,8,8-tetramethyl-5,6,7,8-tetrahydro-2-naphthyl)hydroxyiminomethyl]-2-naphthalenecarboxylic acid,
N-ethyl-6-[(5,5,8,8-tetramethyl-5,6,7,8-tetrahydro-2-naphthyl)hydroxyiminomethyl]-2-naphthalenecarboxamide,
ethyl 6-[(5,5,8,8-tetramethyl-5,6,7,8-tetrahydro-2-naphthyl)aminomethyl]-2-naphthalenecarboxylate,
6-[(5,5,8,8-tetramethyl-5,6,7,8-tetrahydro-2-naphthyl)aminomethyl]-2-naphthalenecarboxylic acid,
6-[(5,5,8,8-tetramethyl-5,6,7,8-tetrahydro-2-naphthyl)carbonyl]-2-naphthalenecarboxylic acid and its methyl ester,
N-ethyl-6-[(5,5,8,8-tetramethyl-5,6,7,8-tetrahydro-2-naphthyl)carbonyl]-2-naphthalenecarboxamide,
6-[(5,5,8,8-tetramethyl-5,6,7,8-tetrahydro-2-naphthyl)carbonyl]-2-naphthalenecarbaldehyde,

6-[(5,5,8,8-tetramethyl-5,6,7,8-tetrahydro-2-naphthyl)carbonyl]-2-naphthalenecarbinol,

N-(2'-hydroxy-2-ethoxyethyl)-6-[(5,5,8,8-tetramethyl-5,6,7,8-tetrahydro-2-naphthyl)carbonyl]-2-naphthalenecarboxamide,

N-(p-hydroxyphenyl)-6-[(5,5,8,8-tetramethyl-5,6,7,8-tetrahydro-2-naphthyl)carbonyl]-2-naphthalenecarboxamide,

N-[1-ethoxycarbonyl-3-(methylthio)propyl]-6-[(5,5,8,8-tetramethyl-5,6,7,8-tetrahydro-2-naphthyl) carbonyl]-2-naphthalenecarboxamide,

N-[1-carboxy-3-(methylthio)propyl]-6-[(5,5,8,8-tetramethyl-5,6,7,8-tetrahydro-2-naphthyl)carbonyl]-2-naphthalenecarboxamide,

6-[(5,6,7,8-tetrahydro-2-naphthyl)carbonyl]-2-naphthalenecarboxylic acid and its methyl ester, and

N-ethyl-6-[(5,6,7,8-tetrahydro-2-naphthyl)carbonyl]-2-naphthalenecarboxamide.

**16.** Compound according to any one of Claims 1 to 13, characterised in that it is 6-[(5,8-methano-5,6,7,8-tetrahydro-2-naphthyl)carbonyl]-2-naphthalenecarboxylic acid and its methyl ester.

**17.** Process for preparing the compounds according to any one of Claims 1 to 16, characterised in that it consists in reacting, in an organic solvent medium under the conditions of the Friedel-Crafts reaction, a halide such as an acid chloride corresponding to the following formula:

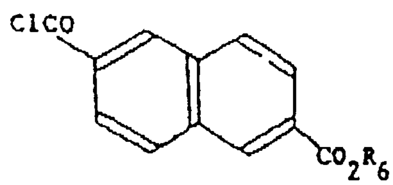

with a naphthalene compound of formula:

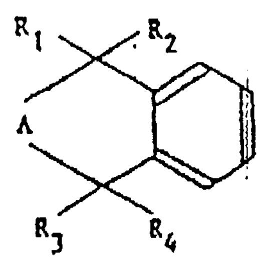

in which compounds:

A and $R_1$ to $R_4$ have the same meanings as those given in Claim 1, $R_6$ being an alkyl radical having from 1 to 20 carbon atoms, and in that, if necessary, the keto ester obtained is saponified to the corresponding keto acid and the said keto acid is subsequently converted to the corresponding amide by the action of an amine of formula

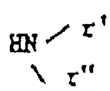

in which:

r' and r'' have the same meanings as those given in Claim 1, or the said keto acid is subsequently converted to a hydroxy acid, to a methano acid, to a hydroxyimino acid, to a methylene acid or to a diol, and the diol is oxidised, where appropriate, to the corresponding alcohol aldehyde or keto aldehyde, or is converted, where appropriate, to an acyloxy or alkoxy derivative.

**18.** Process according to Claim 17, characterised in that the condensation reaction is formed in the presence of anhydrous aluminium chloride in 1,2-dichloroethane at a temperature of between 0 and 25° C, with stirring.

**19.** Process according to Claim 17, characterised in that the preparation of the amide is performed in the presence of N,N'-carbonyldiimidazole.

**20.** Process according to Claim 17, characterised in that the reduction of the keto acid to the corresponding hydroxy acid is performed in the presence of sodium borohydride in THF.

**21.** Process according to Claim 17, characterised in that the alcohol aldehyde and the keto aldehyde are obtained by oxidation of the diol using pyridinium chlorochromate, the said corresponding diol resulting from a reduction reaction of the keto acid in the presence of lithium aluminium hydride.

**22.** Process according to Claim 17, characterised in that the compounds of formula I in which R' = R'' = H are obtained by reduction of the corresponding keto derivatives with zinc in acetic acid in the presence of hydrochloric acid.

**23.** Process according to Claim 17, characterised in that the compounds of formula I in which R' and R'' = methano (= $CH_2$) are obtained by reaction of the corresponding keto compounds with a mixture of triphenylmethylphosphonium bromide and sodium amide.

**24.** Process according to Claim 17, characterised in that the compounds of formula I in which R' and R'' = hydroxyimino (= N-OH) are obtained by reaction of the keto compounds with hydroxylamine hydrochloride in an organic solvent and in the presence of a base.

**25.** Medicinal product, characterised in that it is a compound of formula (I), according to any one of Claims 1 to 16, or is obtained according to any one of Claims 17 to 24.

**26.** Medicinal product according to Claim 25, characterised in that it is administered at a daily dose of approximately 2 µg/kg to 2 mg/kg of body weight.

**27.** Pharmaceutical composition, characterised in that it contains, in a vehicle suitable for enteral, parenteral, topical or ocular administration, at least one compound of the formula (I) according to any one of Claims 1 to 16, or obtained according to any one of Claims 17 to 24.

**28.** Composition according to Claim 27, characterised in that it is presented in a form suitable for topical or ocular administration and contains from 0.0005 to approximately 5% by weight of a compound of formula (I).

**29.** Use of a compound according to any one of Claims 1 to 16, or obtained according to any one of Claims 17 to 24, for the preparation of a pharmaceutical composition intended for the treatment of dermatological, respiratory and also ophthalmological conditions

**30.** Cosmetic composition for body and hair hygiene, characterised in that it contains, in a suitable cosmetic vehicle, at least one compound of the formula (I) according to any one of Claims 1 to 16, or obtained according to anyone of Claims 17 to 24.

**31.** Cosmetic composition according to Claim 30, characterised in that it contains the compound of formula (I) at a concentration of between 0,0005 and 2%, and preferably between 0.01 and 1%, by weight.

**Claims for the following Contracting States: AT, ES, GR**

**1.** Process for preparing a bicyclic naphthalene compound corresponding to the following general formula:

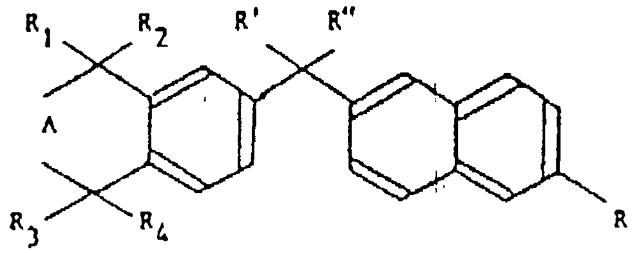

(I)

in which:

A denotes a methylene or dimethylene radical, substituted or unsubstituted with a lower alkyl radical,

$R_1$, $R_2$, $R_3$ and $R_4$, which may be identical or different, denote a hydrogen atom or a lower alkyl radical having from 1 to 6 carbon atoms,

or $R_1$ and $R_3$, taken together, form a methylene or dimethylene bridge, when A denotes a dimethylene radical,

R' denotes a hydrogen atom, an OH radical, an alkoxy radical having from 1 to 4 carbon atoms, an acyloxy radical having from 1 to 4 carbon atoms or an amino radical,

R'' denotes a hydrogen atom or an alkoxy radical having from 1 to 4 carbon atoms,

or R' and R'', taken together, form an oxo (=O), methano (=$CH_2$) or hydroxyimino (=N-OH) radical, and

R denotes a -$CH_2$OH radical or a radical -$COR_5$,

$R_5$ denoting a hydrogen atom, a radical $OR_6$ or a radical

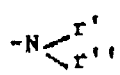

$R_6$ denoting a hydrogen atom, an alkyl radical having from 1 to 20 carbon atoms, a monohydroxyalkyl, polyhydroxyalky, aryl or aralkyl radical, these radicals optionally being substituted, or a residue of a sugar or alternatively a radical

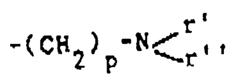

p being 1, 2 or 3 and r' and r'', which may be identical or different, denoting a hydrogen atom, a lower alkyl radical, a monohydroxyalkyl radical optionally interrupted by a hetero atom, a polyhydroxyalkyl radical, an optionally substituted aryl or benzyl radical, an amino acid residue, amino ester residue or amino sugar residue or, taken together, form, with the nitrogen atom to which they are attached, a substituted or unsubstituted heterocycle,

or one of its salts and optical isomers characterised in that it consists in reacting, in an organic solvent medium under the conditions of the Friedel-Crafts reaction, a halide such as an acid chloride corresponding to the following formula:

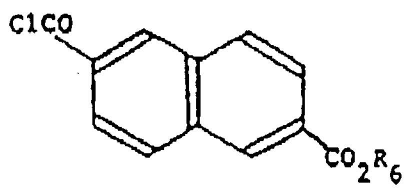

with a naphthalene compound of formula:

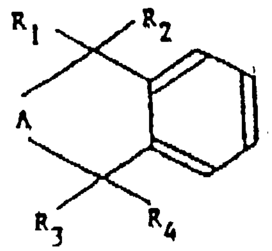

in which compounds:

A and $R_1$ to $R_4$ have the same meanings as those given above, $R_6$ being an alkyl radical having from 1 to 20 carbon atoms, and in that, if necessary, the keto ester obtained is saponified to the corresponding keto acid and the said keto acid is subsequently converted to the corresponding amide by the action of an amine of formula

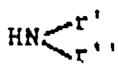

in which:

r' and r'' have the same meanings as those given above, or the said keto acid is subsequently converted to a hydroxy acid, to a methano acid, to a hydroxyimino acid, to a methylene acid or to a diol, and the diol is oxidised, where appropriate, to the corresponding alcohol aldehyde or keto aldehyde, or is converted, where appropriate, to an acyloxy or alkoxy derivative.

**2.** Process according to Claim 1, characterised in that the condensation reaction is formed in the presence of anhydrous aluminium chloride in 1,2-dichloroethane at a temperature of between 0 and 25 °C, with stirring.

**3.** Process according to Claim 1, characterised in that the preparation of the amide is performed in the presence of N,N'-carbonyldiimidazole.

**4.** Process according to Claim 1, characterised in that the reduction of the keto acid to the corresponding hydroxy acid is performed in the presence of sodium borohydride in THF.

**5.** Process according to claim 1, characterised in that the alcohol aldehyde and the keto aldehyde are obtained by oxidation of the diol using pyridinium chlorochromate, the said corresponding diol resulting from a reduction reaction of the keto acid in the presence of lithium aluminium hydride.

**6.** Process according to Claim 1, characterised in that the compounds of formula I in which R' = R'' = H are obtained by reduction of the corresponding keto derivatives with zinc in acetic acid in the presence of sulphuric acid.

**7.** Process according to claim 1, characterised in that the compounds of formula I in which R' and R'' = methano (= $CH_2$) are obtained by reaction of the corresponding keto compounds with a mixture of triphenylmethylphosphonium bromide and sodium amide.

**8.** Process according to Claim 1, characterised in that the compounds of formula I in which R' and R'' = hydroxyimino (= N-OH) are obtained by reaction of the keto compounds with hydroxylamine hydrochloride in an organic solvent and in the presence of a base.

**9.** Use of a compound obtained according to any one of Claims 1 to 8 for the preparation of a pharmaceutical composition intended for the treatment of dermatological, respiratory and also ophthalmological conditions.

**Patentansprüche**

**Patentansprüche für folgende Vertragsstaaten: BE, CH, DE, FR, GB, IT, LI, NL, SE**

**1.** Bicyclische Naphthalinverbindungen der allgemeinen Formel:

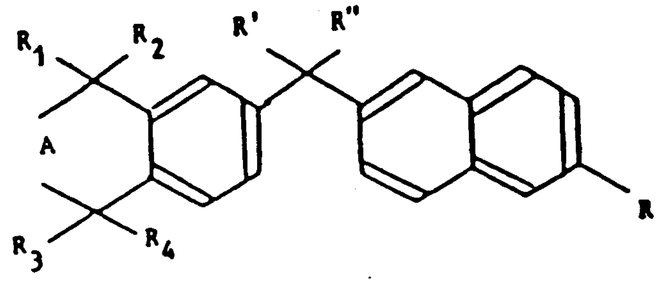

(I)

worin

| | |
|---|---|
| A | einen Methylen- oder Dimethylenrest bedeutet, der unsubstituiert oder mit einem Niedrigalkylrest substituiert ist, |
| $R_1$, $R_2$, $R_3$ und $R_4$ | gleich oder verschieden sind und ein Wasserstoffatom oder einen Niedrigalkylrest mit 1 bis 6 Kohlenstoffatomen bedeuten, oder |
| $R_1$ und $R_3$ | zusammen eine Methylen- oder Dimethylenbrücke bilden, wenn A für einen Dimethylenrest steht, |
| R' | für ein Wasserstoffatom, einen OH-Rest, einen Alkoxyrest mit 1 bis 4 Kohlenstoffatomen, einen Acyloxyrest mit 1 bis 4 Kohlenstoffatomen oder einen Aminorest bedeutet, |
| R'' | ein Wasserstoffatom oder einen Alkoxyrest mit 1 bis 4 Kohlenstoffatomen bedeutet, oder |
| R' und R'' | zusammen einen Oxo(=O)-, Methano(=$CH_2$)- oder Hydroxyimino(=N-OH)-Rest bilden, |
| R | für -$CH_2$OH oder -$COR_5$ steht, |

$R_5$ ein Wasserstoffatom, $OR_6$ oder

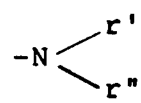

bedeutet,

$R_6$ ein Wasserstoffatom, einen Alkylrest mit 1 bis 20 Kohlenstoffatomen, einen Monohydroxyalkyl-, Polyhydroxyalkyl-, einen gegebenenfalls substituierten Aryl- oder Aralkylrest oder einen Zuckerrest oder

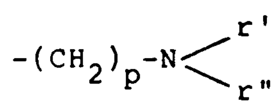

bedeutet,

worin

p für 1, 2 oder 3 steht und r' und r'' gleich oder verschieden sind und für ein Wasserstoffatom, einen Niedrigalkylrest, einen gegebenenfalls durch ein Heteroatom unterbrochenen Monohydroxyalkylrest, einen Polyhydroxyalkylrest, einen gegebenenfalls substituierten Aryl- oder Benzylrest, einen Aminosäurerest, einen Aminoester oder ein Zuckeramin stehen, oder zusammen mit dem Stickstoffatom, an welches sie gebunden sind, einen gegebenenfalls substituierten Heterocyclus bilden,

und die Salze der Verbindungen der allgemeinen Formel (I) sowie deren optische Isomere.

**2.** Verbindungen nach Anspruch 1, dadurch gekennzeichnet, daß die Niedrigalkylreste und Reste mit bis zu 20 Kohlenstoffatomen aus der Gruppe folgender Reste ausgewählt sind: Methyl-, Ethyl-, Propyl-,

Isopropyl-, Butyl-, tert.-Butyl-, 2-Ethylhexyl-, Isooctyl-, Octyl-, Dodecyl-, Hexadecyl- und Octadecylrest.

**3.** Verbindungen nach Anspruch 1, dadurch gekennzeichnet, daß der Monohydroxyalkylrest ein 2-Hydroxyethyl-, 2-Hydroxypropyl- oder 2'-Hydroxy-2-ethoxyethylrest ist.

**4.** Verbindungen nach Anspruch 1, dadurch gekennzeichnet, daß der Polyhydroxyalkylrest ein 2,3-Dihydroxypropyl-, 1,3-Dihydroxy-2-propyl- oder ein Pentaerythritolrest ist.

**5.** Verbindungen nach Anspruch 1, dadurch gekennzeichnet, daß der Alkoxyrest ein Methoxy-, Isopropoxy-, Butoxy- oder tert.-Butoxyrest ist.

**6.** Verbindungen nach Anspruch 1, dadurch gekennzeichnet, daß der Arylrest einen gegebenenfalls durch ein Halogenatom, -OH, $-NO_2$, einen Niedrigalkyl-, Trifluormethylrest oder eine Carbonsäurefunktion substituierten Phenylrest bedeutet.

**7.** Verbindungen nach Anspruch 1, dadurch gekennzeichnet, daß der Aralkylrest ein Benzyl- oder Phenethylrest ist.

**8.** Verbindungen nach Anspruch 1, dadurch gekennzeichnet, daß der Zuckerrest von Glucose, Mannose, Erythrose oder Galaktose abgeleitet ist.

**9.** Verbindungen nach Anspruch 1, dadurch gekennzeichnet, daß der Zuckeraminrest von Glucosamin, Galactosamin, Mannosamin oder Meglumin abgeleitet ist.

**10.** Verbindungen nach Anspruch 1, dadurch gekennzeichnet, daß die Reste r' und r'' zusammen mit dem Stickstoffatom, an welches sie gebunden sind, einen Piperidino-, Piperazino-, Morpholino-, Pyrrolidino- oder 4-(2'-Hydroxyethyl)piperazinorest bilden.

**11.** Verbindungen nach einem der Ansprüche 1 bis 10, dadurch gekennzeichnet, daß sie folgende allgemeine Formel aufweisen:

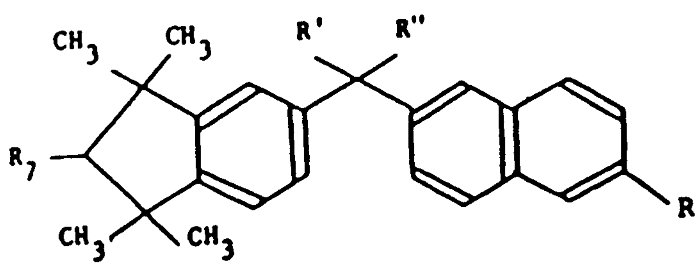

(II)

worin

| | |
|---|---|
| R' | ein Wasserstoffatom oder einen Hydroxylrest bedeutet, |
| R'' | ein Wasserstoffatom bedeutet, oder |
| R' und R'' | zusammen einen Oxo(=O)-Rest bilden, |
| R | für $-CH_2OH$ oder $-COOR_6$ stehen, $R_6$ ein Wasserstoffatom oder einen Alkylrest mit 1 bis 6 Kohlenstoffatomen bedeutet, und |
| $R_7$ | ein Wasserstoffatom oder einen Methylrest bedeutet. |

**12.** Verbindungen nach einem der Ansprüche 1 bis 10, dadurch gekennzeichnet, daß sie folgende allgemeine Formel aufweisen:

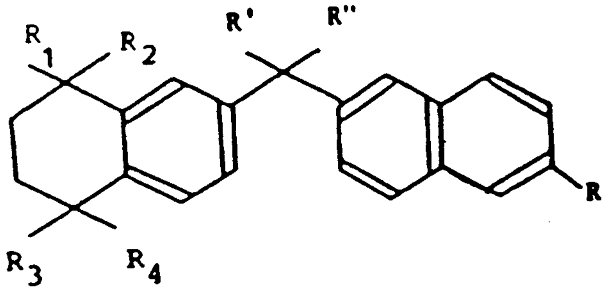

(III)

worin

- $R_1$, $R_2$, $R_3$ und $R_4$ für $-CH_3$ stehen,
- R' ein Wasserstoffatom, einen Hydroxylrest, einen Alkoxyrest mit 1 bis 4 Kohlenstoffatomen, einen Acyloxyrest mit 1 bis 4 Kohlenstoffatomen bedeutet,
- R'' ein Wasserstoffatom bedeutet,
  oder
- R' und R'' zusammen einen Oxo-(=O)- oder Methano(=$CH_2$)-Rest bedeutet, und
- R für $-CH_2OH$ oder $-COR_5$ steht,
  $R_5$ ein Wasserstoffatom, $-OR_6$ oder

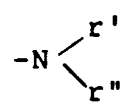

bedeutet,
$R_6$ ein Wasserstoffatom oder einen Alkylrest mit 1 bis 6 Kohlenstoffatomen bedeutet, und r' und r'' ein Wasserstoffatom, einen Alkylrest mit 1 bis 6 Kohlenstoffatomen, 4-Hydroxyphenyl-, 2'-Hydroxy-2-ethoxyethyl- oder 1-Carboxy-3-methylthiopropyl bedeuten.

**13.** Verbindungen nach einem der Ansprüche 1 bis 10, dadurch gekennzeichnet, daß sie folgende allgemeine Formel aufweisen:

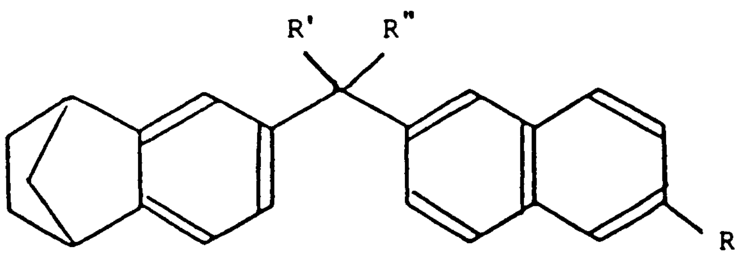

(IV)

worin

- R' und R'' zusammen einen Oxo(=O)-Rest bedeuten,
- R für $-COR_5$ steht,
- $R_5$ für $-OR_6$ oder

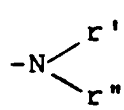

steht,

- $R_6$ für ein Wasserstoffatom oder einen Alkylrest mit 1 bis 6 Kohlenstoffatomen steht, und
- r' und r'' für ein Wasserstoffatom oder einen Alkylrest mit 1 bis 6 Kohlenstoffatomen stehen.

14. Verbindungen nach einem der Ansprüche 1 bis 13, dadurch gekennzeichnet, daß sie aus der Gruppe folgender Verbindungen ausgewählt sind:

6-[(1,1,3,3-Tetramethyl-5-indanyl)carbonyl]-naphthalin-2-methylcarboxylat,
6-[(1,1,3,3-Tetramethyl-5-indanyl)carbonyl]-naphthalin-2-carbonsäure,
6-[(1,1,2,3,3-Pentamethyl-5-indanyl)carbonyl]-naphthalin-2-methylcarboxylat,
6-[(1,1,2,3,3-Pentamethyl-5-indanyl)carbonyl]-naphthalin-2-carbonsäure
6-[(1,1,2,3,3-Pentamethyl-5-indanyl)hydroxymethyl] -naphthalin-2-carbonsäure.

15. Verbindungen nach einem der Ansprüche 1 bis 13, dadurch gekennzeichnet, daß sie aus der Gruppe folgender Verbindungen ausgewählt sind:

N-Ethyl-6-[(1,1,3,3-tetramethyl-5-indanyl) carbonyl]-naphthalin-2-carboxamid,
N-Ethyl-6-[(1,1,2,3,3-pentamethyl-5-indanyl) carbonyl]-naphthalin-2-carboxamid,
N-Ethyl-6-[(1,1,2,3,3-pentamethyl-5-indanyl) hydroxymethyl]-naphthalin-2-carboxamid,

6-[(5,5,8,8-Tetramethyl-5,6,7,8-tetrahydro-2-naphthyl)hydroxymethyl]-naphthalin-2-carbonsäure,
N-Ethyl-6-[(5,5,8,8-tetramethyl-5,6,7,8-tetrahydro-2-naphthyl)hydroxymethyl]-naphthalin-2-carboxyamid,
1-(5,5,8,8-Tetramethyl-5,6,7,8-tetrahydro-2-naphthyl)-1-(6-carboxy-2-naphthyl)methan,
6-[(5,5,8,8-Tetramethyl-5,6,7,8-tetrahydro-2-naphthyl)acetoxymethyl]-naphthalin-2-carbonsäure,
6-[2-(5,5,8,8-Tetramethyl-5,6,7,8-tetrahydro-2-naphthyl)-2-ethenyl]-naphthalin-2-methylcarboxylat,
6-[2-(5,5,8,8-Tetramethyl-5,6,7,8-tetrahydro-2-naphthyl)-2-ethenyl]-naphthalin-2-carbonsäure,
6-[(5,5,8,8-Tetramethyl-5,6,7,8-tetrahydro-2-naphthyl)-hydroxymethyl]-naphthalin-2-carbinol,

6-[(5,5,8,8-Tetramethyl-5,6,7,8-tetrahydro-2-naphthyl)butoxymethyl]-naphthalin-2-carbinol,
6-[(5,5,8,8-Tetramethyl-5,6,7,8-tetrahydro-2, naphthyl)butoxymethyl-]naphthalin-2-carbonsäure,
6-[(5,5,8,8-Tetramethyl-5,6,7,8-tetrahydro-2-naphthyl)hydroxymethyl]-2-naphthaldehyd,
6-[(5,5,8,8-Tetramethyl-5,6,7,8-tetrahydro-2-naphthyl)hydroxyiminomethyl]-naphthalin-2-ethylcarboxylat,
6-[(5,5,8,8-Tetramethyl-5,6,7,8-tetrahydro-2-naphthyl)hydroxyiminomethyl]-naphthalin-2-carbonsäure,
N-Ethyl-6-[(5,5,8,8-tetramethyl-5,6,7,8-tetrahydro-2-naphthyl)hydroxyiminomethyl]-naphthalin-2-carboxamid,
6-[(5,5,8,8-Tetramethyl-5,6,7,8-tetrahydro-2-naphthyl)aminomethyl]-naphthalin-2-ethylcarboxylat,
6-[(5,5,8,8-Tetramethyl-5,6,7,8-tetrahydro-2-naphthyl)aminomethyl]-naphthalin-2-carbonsäure,
6-[(5,5,8,8-Tetramethyl-5,6,7,8-tetrahydro-2-naphthyl)carbonyl]-naphthalin-2-carbonsäure und **deren** Methylester,
N-Ethyl-6-[(5,5,8,8-tetramethyl-5,6,7,8-tetrahydro-2-naphthyl)carbonyl]-naphthalin-2-carboxamid,
6-[(5,5,8,8-Tetramethyl-5,6,7,8-tetrahydro-2-naphthyl)carbonyl]-naphthalin-2-carboxaldehyd,
6-[(5,5,8,8-Tetramethyl-5,6,7,8-tetrahydro-2-naphthyl)carbonyl]-naphthalin-2-carbinol,
N-(2'-Hydroxy-2-ethoxyethyl)-6-[(5,5,8,8-tetramethyl-5,6,7,8-tetrahydro-2-naphthyl)carbonyl]-naphthalin-2-carboxamid,
N-p-Hydroxyphenyl-6-[(5,5,8,8-tetramethyl-5,6,7,8-tetrahydro-2-naphthyl)carbonyl]-naphthalin-2-carboxamid,

N-(1-Ethoxycarbonyl-3-methylthiopropyl)-6-[(5,5,8,8-tetramethyl-5,6,7,8-tetrahydro-2-naphthyl)-carbonyl]-naphthalin-2-carboxamid,
N-(1-Carboxy-3-methylthiopropyl)-6-[5,5,8,8-tetramethyl-5,6,7,8-tetrahydro-2-naphthyl)carbonyl]-naphthalin-2-carboxamid,

6-[(5,6,7,8-Tetrahydro-2-naphthyl)carbonyl]-naphthalin-2-carbonsäure und deren Methylester,
N-Ethyl-6-[(5,6,7,8-tetrahydro-2-naphthyl)carbonyl]-naphthalin-2-carboxamid.

16. Verbindungen nach einem der Ansprüche 1 bis 13, dadurch gekennzeichnet, daß daß es die Verbindung:

6-[(5,8-Methano-5,6,7,8-tetrahydro-2-naphthyl) carbonyl]-naphthalin-2-carbonsäure ist.

Methylester

**17.** Verfahren zur Herstellung von Verbindungen nach einem der Ansprüche 1 bis 16, dadurch gekennzeichnet, daß man in einem organischen Lösungsmittel unter Bedingungen der Friedel-Crafts-Reaktion ein Halogenid wie z.B. ein Säurechlorid der allgemeinen Formel:

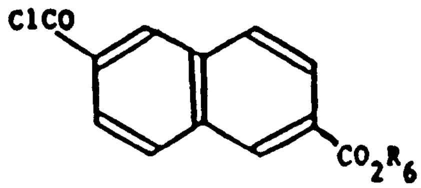

mit einer Naphthalinverbindung der allgemeinen Formel:

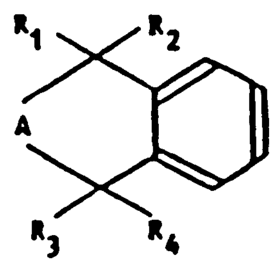

worin
A, $R_1$ bis $R_4$ der in Anspruch 1 gegebenen Definition entsprechen und $R_6$ ein Alkylrest mit 1 bis 20 Kohlenstoffatomen ist, umsetzt und den so erhaltenen Ketoester gegebenenfalls zur entsprechenden Ketosäure verseift und die Ketosäure anschließend durch Umsetzung mit einem Amin der allgemeinen Formel:

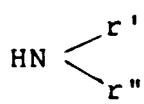

worin
r' und r" der in Anspruch 1 gegebenen Definition entsprechen,
in das entsprechende Amid überführt,
oder die Ketosäure anschließend zur Hydroxysäure, Methanosäure, Hydroxyiminsäure, Methylensäure oder zum Diol umsetzt und das Diol gegebenenfalls zum entsprechenden Alkoholaldehyd oder Ketoaldehyd oxidiert, oder gegebenenfalls in ihre Acyloxy- oder Alkoxy-Derivate überführt.

**18.** Verfahren nach Anspruch 17, dadurch gekennzeichnet, daß die Kondensationsreaktion in Gegenwart von wasserfreiem Aluminiumchlorid in 1,2-Dichlorethan bei einer Temperatur von 0 bis 25 °C unter Rühren durchgeführt wird.

**19.** Verfahren nach Anspruch 17, dadurch gekennzeichnet, daß das Amid in Gegenwart von N,N'-Carbonyldiimidazol hergestellt wird.

**20.** Verfahren nach Anspruch 17, dadurch gekennzeichnet, daß die Reduktion der Ketosäure zur entsprechenden

Hydroxysäure in Gegenwart von Natriumborhydrid in THF durchgeführt wird.

**21.** Verfahren nach Anspruch 17, dadurch gekennzeichnet, daß man den Alkoholaldehyd und den Ketoaldehyd durch Oxidation des Diols mit Pyridiniumchlorchromat oxidiert, wobei man das entsprechende Diol durch Reduktion der Ketosäure in Gegenwart von Lithiumaluminiumhydrid erhält.

**22.** Verfahren nach Anspruch 17, dadurch gekennzeichnet, daß man Verbindungen der allgemeinen Formel (I), worin R' = R" = H ist, durch Reduktion der entsprechenden Ketoderivate mit Zink in Essigsäure in Gegenwart von Chlorwasserstoffsäure erhält.

**23.** Verfahren nach Anspruch 17, dadurch gekennzeichnet, daß man Verbindungen der allgemeinen Formel (I), worin R' und R" für Methano( = $CH_2$) stehen, durch Umsetzung der entsprechenden Ketoverbindungen mit einer Mischung aus Triphenylmethylphosphoniumbromid und Natriumamid erhält.

**24.** Verfahren nach Anspruch 17, dadurch gekennzeichnet, daß man Verbindungen der allgemeinen Formel (I), worin R' und R" für Hydroxyimino( = N-OH) stehen, durch Umsetzung von Ketoverbindungen mit Hydroxylaminhydrochlorid in einem organischen Lösungsmittel in Gegenwart einer Base erhält.

**25.** Medikament, dadurch gekennzeichnet, daß es eine Verbindung der allgemeinen Formel (I) gemäß einem der Ansprüche 1 bis 16 oder hergestellt gemäß einem der Ansprüche 17 bis 24 ist.

**26.** Medikament nach Anspruch 25, dadurch gekennzeichnet, daß es in einer täglichen Dosis von etwa 2 µg/kg bis 2 mg/kg, bezogen auf das Körpergewicht, verabreicht wird.

**27.** Pharmazeutisches Mittel, dadurch gekennzeichnet, daß es in einem geeigneten Träger zur enteralen, parenteralen, topischen oder okularen Verabreichung mindestens eine Verbindung der allgemeinen Formel (I) gemäß einem der Ansprüche 1 bis 16 oder hergestellt gemäß einem der Ansprüche 17 bis 24 enthält.

**28.** Mittel nach Anspruch 27, dadurch gekennzeichnet, daß es in einer zur topischen oder okularen Verabreichung geeigneten Form vorliegt und 0,0005 bis etwa 5 Gew.-% einer Verbindung der allgemeinen Formel (I) enthält.

**29.** Verwendung einer Verbindung gemäß einem der Ansprüche 1 bis 16 oder hergestellt gemäß einem der Ansprüche 17 bis 24, zur Herstellung eines pharmazeutischen Mittels für die Behandlung dermatologischer, respiratorischer sowie ophtalmologischer Erkrankungen.

**30.** Kosmetisches Mittel für die Körper- und Haarpflege, dadurch gekennzeichnet, daß es in einem geeigneten kosmetischen Träger mindestens eine Verbindung der allgemeinen Formel (I) gemäß einem der Ansprüche 1 bis 16 oder hergestellt gemäß einem der Ansprüche 17 bis 24 enthält.

**31.** Kosmetisches Mittel nach Anspruch 30, dadurch gekennzeichnet, daß es eine Verbindung der allgemeinen Formel (I) in einem Konzentrationsbereich von 0,0005 bis 2 Gew.-% und vorzugsweise zwischen 0,01 und 1 Gew.-% enthält.

**Patentansprüche für folgende Vertragsstaaten: AT, ES, GR**

**1.** Verfahren zur Herstellung einer bicyclischen Naphthalinverbindung der allgemeinen Formel:

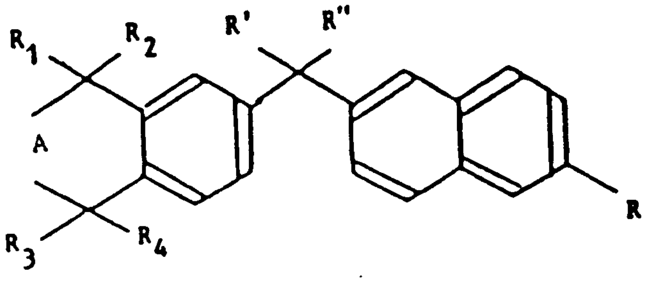

(I)

worin

A einen Methylen- oder Dimethylenrest bedeutet, der unsubstituiert oder mit einem Niedrigalkylrest substituiert ist,

$R_1$, $R_2$, $R_3$ und $R_4$ gleich oder verschieden sind und ein Wasserstoffatom oder einen Niedrigalkylrest mit 1 bis 6 Kohlenstoffatomen bedeuten, oder

$R_1$ und $R_3$ zusammen eine Methylen- oder Dimethylenbrücke bilden, wenn A für einen Dimethylenrest steht,

R' für ein Wasserstoffatom, einen OH-Rest, einen Alkoxyrest mit 1 bis 4 Kohlenstoffatomen, einen Acyloxyrest mit 1 bis 4 Kohlenstoffatomen oder einen Aminorest bedeutet,

R'' ein Wasserstoffatom oder einen Alkoxyrest mit 1 bis 4 Kohlenstoffatomen bedeutet, oder

R' und R'' zusammen einen Oxo(=O)-, Methano(=$CH_2$)- oder Hydroxyimino(=N-OH)-Rest bilden,

R für -$CH_2$OH oder -$COR_5$ steht,
$R_5$ ein Wasserstoffatom, $OR_6$ oder

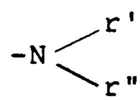

bedeutet,
$R_6$ ein Wasserstoffatom, einen Alkylrest mit 1 bis 20 Kohlenstoffatomen, einen Monohydroxyalkyl-, Polyhydroxyalkyl-, einen gegebenenfalls substituierten Aryl- oder Aralkylrest oder einen Zuckerrest oder

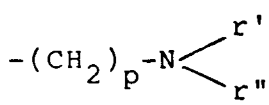

bedeutet,
worin
p für 1, 2 oder 3 steht und r' und r'' gleich oder verschieden sind und für ein Wasserstoffatom, einen Niedrigalkylrest, einen gegebenenfalls durch ein Heteroatom unterbrochenen Monohydroxyalkylrest, einen Polyhydroxyalkylrest, einen gegebenenfalls substituierten Aryl- oder Benzylrest, einen Aminosäurerest, einen Aminoester oder ein Zuckeramin stehen, oder zusammen mit dem Stickstoffatom, an welches sie gebunden sind, einen gegebenenfalls substituierten Heterocyclus bilden,

und von einem Salz oder optischen Isomer davon, dadurch gekennzeichnet, daß man in einem organischen Lösungsmittel unter Bedingungen der Friedel-Crafts-Reaktion ein Halogenid wie z.B. ein Säurechlorid der allgemeinen Formel:

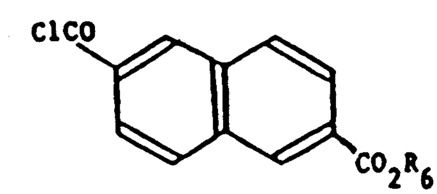

mit einer Naphthalinverbindung der allgemeinen Formel:

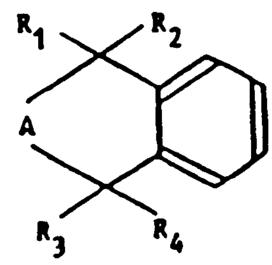

worin
A, $R_1$ bis $R_4$ der in Anspruch 1 gegebenen Definition entsprechen und $R_6$ ein Alkylrest mit 1 bis 20 Kohlenstoffatomen ist, umsetzt und den so erhaltenen Ketoester gegebenenfalls zur entsprechenden Ketosäure verseift und die Ketosäure anschließend durch Umsetzung mit einem Amin der allgemeinen Formel:

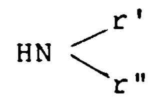

worin
r' und r" der in Anspruch 1 gegebenen Definition entsprechen,
in das entsprechende Amid überführt,
oder die Ketosäure anschließend zur Hydroxysäure, Methanosäure, Hydroxyiminsäure, Methylensäure oder zum Diol umsetzt und das Diol gegebenenfalls zum entsprechenden Alkoholaldehyd oder Ketoaldehyd oxidiert, oder gegebenenfalls in ihre Acyloxy- oder Alkoxy-Derivate überführt.

**2.** Verfahren nach Anspruch 1, dadurch gekennzeichnet, daß die Kondensationsreaktion in Gegenwart von wasserfreiem Aluminiumchlorid in 1,2-Dichlorethan bei einer Temperatur von 0 bis 25 °C unter Rühren durchgeführt wird.

**3.** Verfahren nach Anspruch 1, dadurch gekennzeichnet, daß das Amid in Gegenwart von N,N'-Carbonyldiimidazol hergestellt wird.

**4.** Verfahren nach Anspruch 1, dadurch gekennzeichnet, daß die Reduktion der Ketosäure zur entsprechenden Hydroxysäure in Gegenwart von Natriumborhydrid in THF durchgeführt wird.

**5.** Verfahren nach Anspruch 1, dadurch gekennzeichnet, daß man den Alkoholaldehyd und den Ketoaldehyd durch Oxidation des Diols mit Pyridiniumchlorchromat oxidiert, wobei man das entsprechende Diol durch Reduktion der Ketosäure in Gegenwart von Lithiumaluminiumhydrid erhält.

**6.** Verfahren nach Anspruch 1, dadurch gekennzeichnet, daß man Verbindungen der allgemeinen Formel (I), worin R' = R" = H ist, durch Reduktion der entsprechenden Ketoderivate mit Zink in Essigsäure in Gegenwart von Schwefelsäure erhält.

**7.** Verfahren nach Anspruch 1, dadurch gekennzeichnet, daß man Verbindungen der allgemeinen Formel (I), worin R' und R" für Methano(= $CH_2$) stehen, durch Umsetzung der entsprechenden Ketoverbindungen mit einer Mischung aus Triphenylmethylphosphoniumbromid und Natriumamid erhält.

**8.** Verfahren nach Anspruch 1, dadurch gekennzeichnet, daß man Verbindungen der allgemeinen Formel (I), worin R' und R" für Hydroxyimino(= N-OH) stehen, durch Umsetzung von Ketoverbindungen mit Hydroxylaminhydrochlorid in einem organischen Lösungsmittel in Gegenwart einer Base erhält.

**9.** Verwendung einer Verbindung erhältlich gemäß einem der Ansprüche 1 bis 8 zur Herstellung eines pharmazeutischen Mittels für die Behandlung dermatologischer, respiratorischer sowie ophtalmologischer Erkrankungen.